Europäisches Patentamt

European Patent Office

Office européen des brevets

(19)

(11) Publication number:

**0 169 146**
**A2**

(12) **EUROPEAN PATENT APPLICATION**

(21) Application number: 85401465.1

(22) Date of filing: 17.07.85

(51) Int. Cl.⁴: **C 12 N 15/00,** C 12 N 5/00,
C 12 P 21/00
// (C12P21/00, C12R1:91)

(30) Priority: 20.07.84 US 632785
10.04.85 US 721735

(43) Date of publication of application: 22.01.86
Bulletin 86/4

(84) Designated Contracting States: **AT BE CH DE FR GB IT
LI LU NL SE**

(71) Applicant: **MERCK & CO. INC., 126, East Lincoln Avenue
P.O. Box 2000, Rahway New Jersey 07065 (US)**

(72) Inventor: **Colonno, Richard J., 17 Brookdale Drive, New
Britain Pennsylvania 18910 (US)**
Inventor: **Mitzutani, Satoshi, 854 Village Circle, Blue Bell
Pennsylvania 19422 (US)**

(74) Representative: **Warcoin, Jacques et al, Cabinet
Régimbeau 26, avenue Kléber, F-75116 Paris (FR)**

(54) **Human rhinovirus RNA and proteins.**

(57)    DNA fragments encoding all the structural and non-
structural proteins and untranslated RNA genome regions of
humanrhinovirus type 14 have been molecularly cloned and
their nucleotide sequence determined. The composition and
sequence of the entire 7,212 nucleotide long genome RNA
have been determined. Monoclonal antibodies have been
produced which block the binding of virus to susceptible cells
or which neutralize the infectivity of the virus.

EP 0 169 146 A2

- 1 -   17112Y

## HUMAN RHINOVIRUS RNA AND PROTEINS

### BACKGROUND OF THE INVENTION

Human rhinoviruses (HRV) belong to the picornavirus family and contain at least 115 antigenically distinct serotypes. The picornavirus family also includes poliovirus and hepatitis A virus. Rhinoviruses are the most important common cold viruses to be discovered. The name "rhinovirus" reflects the prominent nasal involvement seen in infections with these viruses. The number of antigenically distinct rhinoviruses is so large that a person can be infected with a different rhinovirus each year and still not experience all of the known types in a life time.

HRV exist as a capsid structure (20-40 nm) with cubic symmetry utilizing 60 identical subunits composed of four peptide chains. The virions are ether resistant and acid labile. HRV contains an infectious, single-stranded genomic RNA approximately 7,200 bases in length. Upon entrance into a susceptible cell, the RNA is immediately translated into a 240,000 mw polyprotein using host cell translational machinery. This polyprotein is subsequently processed by both cellular and viral encoded proteases to yield the structural and non-structural proteins needed for completion of the infectious cycle.

Limited research has been performed on HRV because of the numerous serotypes involved and the poor growth and purification procedures for HRV used in the past. In addition, past procedures utilized in direct RNA and protein sequencing were very laborious and time consuming. However, recent technological advances in cloning and sequencing of nucleic acid has allowed molecular characterization of HRV. Using these techniques, the entire genomic RNA and corresponding amino acid sequence of a HRV could be determined. This information would define significant target areas of the virus (i.e., proteolytic cleavage sites) for which specific antiviral agents could be developed. It has also enabled the isolation of DNA fragments which could be used as probes to determine common genomic regions

0169146

2784P/0973A — 3 — 171121A

among the many serotypes of rhinoviruses. This would allow construction of a genomic map of common and unique sequences within the rhinovirus family.

OBJECTS OF THE INVENTION

It is an object of the present invention to provide a method for isolating and cloning of DNA representative of the genomic RNA of human rhinoviruses. Another object is to determine the nucleotide and derived amino acid sequences of HRV type 14 genomic RNA. A third object is to determine the amino acid sequence of precursor proteins utilized during polyprotein processing. Yet another object is to provide a method for producing HRV proteins or subunits thereof by expression of cloned DNA in an appropriate host. Another object is to provide HRV proteins or subunits thereof which will be useful as immunogens to raise antibodies to HRV. Still another object is to provide a vector containing DNA representative of HRV type 14 genomicORNA or part thereof. A further object is to provide for construction of an infectious cDNA plasmid capable of yielding infectious virus particles when placed in an appropriate host. Another object is to produce a monoclonal antibody capable of blocking the binding of a major group of rhinoviruses. A further object is to produce monoclonal antibodies to rhinovirus structural proteins (VP1, VP2 and VP3) that neutralize the infectivity of the virus. These and other objects of the present invention will be apparent from the following description.

0169146

SUMMARY OF THE INVENTION

DNA fragments encoding the entire 9212 nucleotides of HRV 14 genome RNA have been molecularly cloned and their nucleotide sequence completely determined. Eight monoclonal antibodies have been isolated which neutralize infections HRV-14 virions. In addition, a mouse monoclonal antibody was isolated which specifically blocked the attachment of at least 75 antigenically distinct serotypes of HRVs to cellular receptors.

DETAILED DESCRIPTION

Plaque purified human rhinovirus type 14 was grown and purified by isopycnic banding in a metrizamide (2-[3-acetamido-5-N-methylacetamido-2,4,6-triiodobenzamido]-2-deoxy-D-glucose) gradient. Purified virus was disrupted with detergents and capsid proteins digested with proteinase K. Genomic RNA was isolated by oligo (dT) cellulose chromatography and purified by velocity sedimentation in a sucrose gradient. RNA sedimenting at 35S, relative to ribosomal RNA markers, was collected and ethanol precipitated.

Recovered genomic RNA was used as a template for synthesis of its complementary strand to produce a double stranded DNA molecule corresponding to at least a portion of the original genome RNA. The double stranded DNA was modifed to provide "sticky ends" and was placed into a bacterial plasmid pBR 322. Prokaryotic organisms were exposed to the resulting vector and those which stably incorporated

0169146

2784P/0973A — 5 — 171121A

the vector were identified and isolated.

Vector DNA was extracted from these hosts and this material was characterized. Vector DNA was first analyzed for size of the HRV cDNA insert and vectors containing the largest inserts mapped for their relationship to the viral RNA genome and each other. Selected vectors containing DNA sequences representing the entire length of the HRV genome RNA were sequenced using a known sequencing method. Fragments of these vectors were radioactively labeled and isolated prior to sequencing. By overlapping the sequences obtained from these fragments, the entire HRV 14 sequenece was determined. Subsequent conversion of the DNA sequence to RNA and protein was made by computer. Determination of the amino acid sequence allows one to determine the structure of the virus and to identify its structural and non-structural proteins. Knowledge of the viral structure allows inferences to be made as to likely viral receptor sites, proteolytic cleavage sites, and neutralization sites of the virus. The cloned DNA is available for genetic analysis and modification and for use in the development of antiviral compounds. Expression in suitable hosts would yield large quantities of viral proteins for development of antiviral compounds or subunit vaccines.

The use of recombinant DNA techniques is described in many published articles, for example, Maniatis _et al_, Molecular Cloning, A Laboratory Manual, Cold Spring Harbor, New York, 1982, the disclosure of which is hereby incorporated by reference.

In order to generate monoclonal antibodies that neutralize the infectivity of HRV 14 or that block the binding of many HRV serotypes to the virus receptor on susceptible cells, mice were immunized with either purified virions or whole cells and cell membranes enriched for HRV receptor sites, e.g., HeLa cells. Hybridomas were produced following fusion of spleen cells of immunized mice with mouse myeloma cells. From the resulting hybridoma cell lines eight monoclonal antibodies were isolated which are capable of neutralizing HRV-14. In addition, a monoclonal antibody was selected that was capable of blocking the attachment of at least 75 different serotypes of HRV to receptor sites on susceptible cells. The specific serotypes which were unable to cause infection in the presence of the monoclonal antibody as well as those serotypes which were able to cause infection in the presence of the monoclonal antibody of the present invention are shown in Table I.

As a result of these experiments it has been determined that human rhinoviruses consist of a major group and a minor group. The major group is neutralized or rendered incapable of causing infection by the monoclonal antibody while the minor groups seems unaffected by the monoclonal antibody.

## TABLE I

CHARACTERIZATION OF MONOCLONAL ANTIBODY
TO HRV CELLULAR RECEPTOR

ANTIBODY TYPE: IgG-1

### PROTECTION AGAINST INFECTION BY:

| | | | | | | | |
|---|---|---|---|---|---|---|---|
| HRV-3 | HRV-12 | HRV-21 | HRV-33 | HRV-42 | HRV-57 | HRV-67 | HRV-77 |
| HRV-4 | HRV-13 | HRV-22 | HRV-34 | HRV-46 | HRV-58 | HRV-68 | HRV-78 |
| HRV-5 | HRV-14 | HRV-23 | HRV-35 | HRV-48 | HRV-59 | HRV-69 | HRV-79 |
| HRV-6 | HRV-15 | HRV-24 | HRV-36 | HRV-50 | HRV-60 | HRV-70 | HRV-80 |
| HRV-7 | HRV-16 | HRV-25 | HRV-37 | HRV-51 | HRV-61 | HRV-71 | HRV-81 |
| HRV-8 | HRV-17 | HRV-26 | HRV-38 | HRV-52 | HRV-63 | HRV-72 | HRV-83 |
| HRV-9 | HRV-18 | HRV-27 | HRV-39 | HRV-54 | HRV-64 | HRV-73 | HRV-84 |
| HRV-10 | HRV-19 | HRV-28 | HRV-40 | HRV-55 | HRV-65 | HRV-74 | HRV-85 |
| HRV-11 | HRV-20 | HRV-32 | HRV-41 | HRV-56 | HRV-66 | HRV-75 | HRV-86 |
| | | | | | | HRV-76 | HRV-88 |
| | | | | | | | HRV-HANKS |

### NO PROTECTION AGAINST INFECTION BY:

| | | | |
|---|---|---|---|
| HRV-1A | HRV-29 | HRV-44 | HRV-62 |
| HRV-1B | HRV-30 | HRV-47 | HRV-87 |
| HRV-2 | HRV-31 | HRV-49 | |

2784P/0973A                  - 8 -                    17112IA

It has also been found that the monoclonal antibody of the present invention binds strongly to susceptible cells and possesses greater avidity for the cell as it actually displaces the major group of human rhinoviruses bound to the cell. This characteristic of the antibody of the present invention is shown in Table II. Human rhinoviruses labeled with $S^{35}$ methionine were bound to susceptible cell membranes *in vitro* and unbound virus was removed by washing.

Increasing amounts of the monoclonal antibody of the present invention were added to the membranes and the amount of bound virus that was released was measured.

The monoclonal antibody of the present invention is useful to prevent HRV infection or to ameliorate the duration and severity of infection. Treatment of susceptible cells with the monoclonal antibody of the present invention renders the cells resistant to viral infection for a period of up to 12 hours following removal of excess antibody. The monoclonal antibody may be prepared in a suitable topical formulation for administration in the form of drops or as a spray. The dosage may range of from about 1.5 to about 150 μg given at intervals of from about 2 hours to about 12 hours, and more usually in a range of from about 5 to about 25 μg given at intervals of about 4, 8 or 12 hours. A typical formulation employs the monoclonal antibody of the present invention in PBS containing a stabilizer, e.g., 20% SPGA.

## TABLE II

The following examples illustrate the present invention without, however, limiting the same thereto. The disclosure of each reference mentioned in the following examples is hereby incorporated by reference.

## EXAMPLE 1
## Virus Growth and Purification

HRV 14 was obtained from the American Type Culture collection (Rockville, MD), ATCC VR-284 and plaque purified by standard techniques (Yin et al., J. Virol. 12:108-113, 1973). Plaque purified HRV was propagated on HeLA R19 cells, a subclone of HeLa cells derived by standard procedures (Coller t al., Hybridoma 2:91-96, 1983). Cells were routinely propagated in growth medium (GM): McCoy's 5A medium containing 10% fetal calf serum, 30 mM $MgCl_2$ and 20 units/ml penicillin and 20 µg/ml streptomycin. HeLa R19 monolayers were infected at a MOI of 1 and harvested when cells detached freely from the flask surface (usually 16-24 hours). The cells suspension was frozen and quickly thawed to release virus particles from the cells. After clarification by centrifugation at slow speed (4000 x g for 5 minues at 4°C), Polyethylene glycol 6000 (PEG-6000) and NaCl were added to concentrations of 7% and 2.2% respectively and the mixture stirred at 4°C for 4-16 hours. The precipitated virus was recovered by centrifugation (10,500 x g for 15 minutes at 4°C) and resuspended in 10 mM R-buffer (a solution containing 10 mM tris-HCl pH 7.5, 1 mM EDTA [ethylenediamine tetraacetic acid], 0.2 M NaCl, 50 mM $MgCl_2$ and 10% glycerol). Sodium deoxycholate and polyoxyethylene (9) octaphenol were added to concentrations of 0.3% and 0.6%, respectively, for 30 minutes on ice and the suspension clarified by centrifugation (4000 x $\underline{g}$ for 5 minutes at 4°C). The supernatant liquid (7.5 ml) was layered over a 5 ml linear density gradient of 40-60% (w/v) metrizamide in R-buffer lacking the glycerol. Isopycnic banding of the virus sample was achieved by centrifugation for 24 hours at 150,000 x $\underline{g}$ in a Beckman SW40 rotor. Virus bands were generally the densest of the visible bands in the gradient and were harvested manually by bottom puncture. After dilution, 3-fold in R-buffer the virus was repelleted by centrifugation at 200,000 x $\underline{g}$ for 2 hours.

EXAMPLE 2

Genomic RNA Isolation

HRV 14 purified from forty 150 $cm^2$ flasks of HeLa R19 cells was resuspended in 0.9 ml 10 mM Tris-HCl, 1 mM EDTA, 0.3% SDS (sodium dodecyl sulfate), pH 7.5. Proteinase K was added to a final concentration of 0.5 mg/ml and the viral proteins digested for 30 minutes at 37°C. The digested mixture was heated to 95°C for 1 minute and then quick chilled in an ice bath. NaCl was added to 0.5 M and the solution passed through a 1 cm x 0.7 cm column of oligo (dT) cellulose column (Collaborative Research, Lexington, Mass.) Genomic RNA retained on the column was eluted with $H_2O$ and precipitated at

-20°C with ethanol. Pelleted RNA was resuspended in 0.3 ml of a solution containing 10 mM Tris, 1 mM EDTA, 0.5% SDS, pH 7.5, and layered on a 12 ml preformed gradient from 15% w/w to 30 w/w sucrose in the same buffer but containing 0.1 M NaCl. RNA was sedimented at 23,000 RPM for 17 hours using a Beckman SW40 rotor. A parallel gradient containing ribosomal 18S and 28S RNAs (Bethesda Research Labs., Bethesda, MD) was centrifuged at the same time. Gradients were fractionated (0.4 ml) by an ISCO gradient fractionator and the $OD_{260}$ of each fraction measured. A major peak of viral RNA sedimenting at 35S, relative to the position of 18S and 28S in the parallel marker gradient, was pooled and precipitated twice with ethanol at -20°C.

## EXAMPLE 3

### cDNA Synthesis

Pelleted 35S RNA was resuspended in 8 µl $H_2O$ and boiled for 30 seconds, then quickly chilled in an ice bath. The following were then added: 9.5 µl of 1 mg/ml solution of oligo($dT_{9-12}$) primer (Collaborative Research)), 5 µl of 1M Tris-HCl, pH 8.3, 2 µl of 250 mM $MgCl_2$, 7 µl of 1M KCl, 1 µl of 20 mM DTT (dithiothreitol), 2 µl of RNasin (Promega Biotech, Madison, WI), 2.5 µl each of 20 mM dCTP, 20 mM dGTP, 20 mM dTTP and 10 mM dATP, 12 µl of [$\alpha^{32}$P] labeled dATP (115 uCi) (Amersham). The reaction was preincubated on ice for 10 minutes, followed by the addition of 3 µl of reverse transcriptase (40 units) (Life Sciences, Fla.). The reaction was incubated at 42°C for 2 hours and then stopped by the addition of 5 µl of 0.5M EDTA and 5 µl of 5M NaOH. Following an additional 30 minutes at 37°C, the synthesized cDNA was isolated by cellulose (Whatman CF-11) column chromatography (Franklin, P.N.A.S. 55: 565, 1966) and ethanol precipitated at -20°C.

The cDNA was pelleted and resuspended in 40 µl of $H_2O$ and 50 µl of a solution containing 0.2M Hepes pH 6.9, 20 mM $MgCl_2$, 5 mM DTT, 0.14M KCl, and 1 mM each of dATP, dCTP, dGTP, dTTP. The second DNA strand was then synthesized by the addition of 10 µl of the large fragment of DNA polymerase I, 50 units (New England Biolabs). The reaction mix was incubated for 16 hours at 15°C and stopped by the addition of 2 µl of 0.5M EDTA. The double stranded DNA was phenol/chloroform extracted and precipitated with ethanol. To aid in the completion of the second strand synthesis, the double stranded DNA was resuspended in 30 µl of $H_2O$, 5 µl of 1M Tris pH 8.3, 7 µl of 1M KCl, 2 µl of 250 mM $MgCl_2$, 2.5 µl each of 20 mM dCTP, dGTP, dTTP and 10 mM dATP, 2 µl of 2-mercaptoethanol, and 2 µl (30 units) of reverse transcriptase. The reaction was incubated at 42°C for 1 hour and stopped by the addition of 2 µl of 0.5M EDTA. The double stranded cDNA was ethanol precipitated at -20°C.

EXAMPLE 4

Conversion of cDNA "Hairpin Structure" to Linear Form

The order to insert cDNA into plasmid vectors for cloning, it is necessary to cleave the "hairpin structure" formed during synthesis into a linear form possessing accessible termini at each end of the molecule. This was carried out as follows; the cDNA, previously synthesized in Example 3, was resuspended in 79 µl $H_2O$, and 20 µl of a solution containing 1M NaCl, 0.25M NaAcetate pH 4.5, 5 mM $ZnSO_4$ and 2.5% v/v glycerol. $S_1$ nuclease, 290 units, (PL Biochemicals, Piscataway, N.J.) was added and the reaction incubated 30 minutes at 37°C. The reaction was stopped by the addition of 2 µl of 0.5M EDTA, phenol/chloroform extracted and the DNA purified by chromatography on a cellulose column (Whatman CF-11) as described by Franklin, supra.

EXAMPLE 5

Addition of Oligo dC to 3' Ends of cDNA

To allow insertion of cDNA into oligo deoxyguanosine (dG) "tailed" pBR322, oligo deoxycytidine (dC) "tails" were added to the cDNA from Example 4. Following ethanol precipitation, the cDNA (approximately 2 µg) was resuspended in 149 µl of $H_2O$, 40 µl of 1M potassium cacadylate buffer pH 7.0, 4 µl of 0.1M $CoCl_2$, 2 µl of 0.1M 2-mercaptoethanol, 1 µl of 0.1 M [$^3H$] dCTP (150 µCi) and 4 µl (60 units) terminal deoxytransferase (PL Biochemicals). The reaction was incubated at 37°C and aliquots removed at 1 minute (80 μl), 2 minutes (80 μl), and 3 minutes (40 μl) and pooled together on ice.  The "tailed" cDNA was ethanol precipitated at -20°C.

## EXAMPLE 6
### Vector/cDNA Annealing and Cell Transformation

To insert the tailed cDNA from Example 5 into pBR322, 25 ng of pBR322 oligo dG cloning vector (BRL, Bethesda, MD) was annealed with 13, 26, and 39 ng of oligo dC tailed cDNA in 40 μl of a solution containing 10 mM Tris-HCl pH 7.5, 1 mM EDTA and 0.1M NaCl at 65°C for 5 minutes and 56°C for 90 minutes. After annealing, each reaction mix was cooled to 0°C and added to 200 μl calcium treated E. coli strain RRI (Maniatis et al, op. cit., p. 250).  After a 30 minute incubation on ice, the reaction was heated for 2 minutes at 42°C, 1 ml of L broth (Bacto tryptone 10 g, Bacto yeast extract 5 g, and NaCl 5 g, brought up to 1 l with distilled water) added, and the transformation reaction incubated at 37° for 30 minutes.  Each transformation reaction was plated (250 μg) onto 4 agar petri plates containing tetracycline (15 μg/ml) (Maniatis et al., op. cit., p. 70-72).  Each plate yielded approximately 30 colonies after 16 hours of incubation at 37°C.

## EXAMPLE 7
### Characterization of Selected Clones

Colonies (354) from Example 6 were picked with a toothpick and used to inoculate 200 µl of L broth containing 12 µg/ml tetracycline in 96 well serological plates. Following a 16 hour incubation at 37°C, 20 µl of the new growth culture was used to inoculate 2 ml L broth containing 12 ng $Na_2HPO_4$, 6 ng $KH_2PO_4$, 1 ng NaCl, 2 ng $NH_4Cl$, and 4 µg glucose. After shaking at 37°C for 16 hours, 0.5 ml was frozen after adding glycerol to 15% and stored at -70°C. The remaining 1.5 ml was pelleted 1 minute at 12,000 x g and the supernatant liquid discarded. The pellet was resuspended in 0.15 ml of a solution containing 8% w/v sucrose, 50 mM EDTA, 50 mM Tris-HCl pH 8.0, 5% v/v polyoxyethylene (9) octaphenol and 0.6 mg/ml lysozyme and incubated 5 minutes at 25°C. The bacteria were then placed in a boiling water bath for 40 seconds and immediately plunged into an ice bath. Following centrifugation for 15 minutes at 12,000 x g, the pellet was removed with a toothpick and discarded. An equal volume of isopropanol was added to the supernatant liquid and the solution mixed and placed at -20°C for 10 minutes. After pelleting for 5 minutes at 12,000 x g, the supernatant liquid was removed and the pellet washed with 70% v/v ethanol and dried. The resulting plasmid DNA was resuspended in 50 µl $H_2O$ and stored at -20°C.

To determine the size of each cDNA insert, 10 µl of each plasmid preparation was digested with the restriction endonuclease Pst I (Boehringer Mannheim, Indianapolis IN) as described by the manufacturer. The digests were analyzed by electophoresis in a 1% agarose gel as described (Maniatis et al., op. cit., p. 150). Three cDNA clones, designated 7, 57, and 186, were selected based on size ( 2000 bp) and colony hybridization studies (Grunstein and Hogness, Proc. Nat. Acad. Sci. U.S.A. 72:3691-3965, 1975) which indicated that 7 (3200 bp) and 186 (3200 bp) did not overlap and that 57 (2000 bp) bridged both 7 and 186.

## EXAMPLE 8
### Construction of Deletion Subclones

Twenty micrograms of clones 7 and 186 (Example 7) were digested with 20 units of restriction endonuclease Pst I in a total volume of 30 µl as described by the manufacturer. After separation of plasmid DNA from viral cDNA on a 1% low melt agarose gel (FMC Corporation, Rockland, ME), each cDNA insert was extracted by the method of Tautz and Renz (Anal. Biochem. 132:14-19, 1983) 20 µg bacterial plasmid pUC9 DNA (Vieiera et al., Gene 19: 259-268, 1982) was digested with Pst I as described above and mixed with 30 µl of a 1M solution of Tris-HCl pH 8.0 containing 20 units of alkaline phosphatase (Boehringer Mannheim) for 2 hours at 65°C. The mixture was extracted twice with phenol/chloroform and the pUC9 DNA ethanol precipitated. One µg (0.5 p mole) of both insolated clone 7 and 186 inserts were ligated separately to 2.8 µg (1.0 p mole) of digested  pUC9 DNA with 2.5

units of $T_4$ DNA ligase (BRL, Gaithersburg, MD) in 10 µl of a solution containing 5 mM Tris-HCl pH 8.0, 10 mM $MgCl_2$, 20 mM DTT, 1 mM ATP, and 0.001% polyoxyethylene (9) octaphenol at 15°C for 2 hours.

E. coli strain HB101 (Gene 6:23-28, 1979) was transformed with each of the above ligation mixtures and transformants selected on agar plates containing 100 µg/ml ampicillin. The orientation of each insert in pUC9 plasmid was determined in relation to the orientation of the LAC gene. An "A" orientation of the insert is the same orientation as the LAC gene and a "B" is the reverse. Two hundred µg of pUC9 (7A) and pUC9 (186B) were linearized with DNase I (Boehringer Mannheim) in the presence of 1 mM $MnCl_2$ and DNAs precipitated with PEG 6000 according to the method of Hong (J. Mol. Bio. 158:539-549, 1982). Full length DNA was separated by gel electrophoresis (described above) and recovered from the agarose by the method of Wieslander (Anal. Biochem. 98:305, 1979). Ten µg of linearized pUC9 (7A) or pUC9 (186B) were digested with 20 units of restriction endonuclease Sal I (Boehringer Mannheim) in 40 µl as described by the manufacturer. The reaction mixtures were then heated to 65°C for 5 minutes and 3 µl 1.0M Tris-HCl pH 7.5, 1.5 µl 0.25M $MgCl_2$, 12 µl of a solution containing 0.1 mM each of dATP, dCTP, dGTP, and dTTP, 2.5 µl $H_2O$, and 1 µl (2.5 units) "large fragment" of DNA polymerase (Boehringer Mannheim) were added and incubated at 37°C for 30 minutes. Following an incubation at 65°C for 10 minutes, the DNA was precipitated by adding 60 µl of 10% PEG-6000 and 1.25M NaCl and incubated on ice for 1.5 hours. The DNA was collected by centrifugation (12,000 x g) for 10 minutes and dissolved in 50 µl of a solution containing 10 mM Tris-HCl pH 7.5, 1 mM EDTA and 10 mM NaCl. The DNA was then extracted by phenol/chloroform and ethanol precipitated for 10 minutes at -70°C. The final precipitate was dissolved in 10 µl of a solution containing 10 mM Tris-HCl pH 7.5, 1 mM EDTA and 10 mM NaCl, and mixed with 1.1 µl of a solution containing 0.5M Tris-HCl pH 8.0, 0.1M $MgCl_2$, 0.2M DTT, 0.01M ATP, and 0.01% polyoxyethylene (9) octaphenol, and 1 µl (10 units) $T_4$ DNA ligase (New England Nuclear). The mixture was incubated at 15°C for 4 hours and used to transform E. coli strain HB101 cells as described above. From 10 µg of pUC9 (7A) and pUC9 (186B), 10,000 and 17,000 transformants were obtained, respectively.

Deletion clones (transformants) were screened as described in Example 7 and the size of each DNA insert was determined by restriction endonuclease digestion with BAM HI [pUC9 (186B) clones] and with Pst I and Bam HI [pUC9 (7A) clones].

## EXAMPLE 9

### Nucleic Acid Sequencing of cDNA Inserts

Digestion of clones 7, 57, and 186 and their subset of deletion clones (Example 8) with various commercially available restriction enzymes generated a subset of overlapping DNA fragments. These fragments were radioactively labeled one of two ways.

When restriction enzyme digestion resulted in a 5' overhang in double stranded DNA (i.e., XBA I), 25 µg of the DNA were labeled by resuspending in 80 µl of a solution containing 50 mM NaCl, 6 mM Tris-HCl pH 7.5, 6 mM $MgCl_2$, 6 mM 2-mercaptoethanol and 150-200 uCi $[\alpha^{32}P]$ deoxynucleotide triphosphate representing the first nucleotide to be specified by the overhang, and 5 units of "large fragment" of DNA polymerase I (Boehringer Mannheim) and incubated at 15°C for 2 hours.  Alternatively, when the restriction enzyme digestion resulted in a 3' overhang in the DNA (i.e. Pst I), the DNA was labeled at the 3' end by resuspension in 40 µl of a solution containing 0.2M K cacodylic acid ph 7.0, 2 mM $CaCl_2$, 2 mM 2-mercaptoethanol, 250 uCi $[\alpha^{32}P]$ dd ATP (Amersham, Chicago, IL) and 20 units terminal deoxytransferase (PL Biochemicals) and incubated 1 hour at 37°C.

Both reactions were stopped by the addition of 4 µl of 0.5M EDTA and labeled DNA separated from unincorporated triphosphates by chromatography through an "Elutip" (Schleicher and Schuell, Keene, NH) using the supplier's protocol.  Purified DNA was precipitated with ethanol and recut with a second restriction endonuclease which cut asymmetrically between the radioactively labeled ends, generating at least 2 fragments for each labeled fragment.  The cut fragments were separated by electrophoresis in a 5% (w/v) acrylamide gel [15 x 15 x 0.015 cm] containing 42 ml of 29% w/v acrylamide (Bio Rad), 1% w/v N,N'-bis-acrylylcystamine (Bio Rad), 0.1% w/v ammonium persulfate, 0.38% N,N,N',N'-tetramethylenediamine (Bio Rad), 90 mM Tris base, 140 mM boric acid, and 1 mM EDTA] at 20 mA for 3 hours. The gel was stained in 200 ml of a solution containing 10 mM Tris-HCl pH 7.5, 1 mM EDTA and 1 µg/ml ethidium bromide for 30 minutes and DNA bands visualized by UV light. Appropriate bands were excised and the DNA recovered by dissolving the acrylamide gel slice in 1 ml of a solution containing 0.2M NaCl, 20 mM Tris pH 7.4, 1 mM EDTA and 40 mM 2-mercaptoethanol for 15 minutes at 25°C. The dissolved gel solution was then chromatographed through an "Elutip" (see above) and recovered DNA ethanol precipitated.

## EXAMPLE 10
### Sequence Determination

DNA fragments, labeled with [$^{32}$P] at only one end as described in Example 9, were sequenced using the chemical DNA sequencing methods described by Maxam and Gilbert (Methods in Enzymology, Vol. 65. 1980). Synthetic deoxynucleotide primers and Sanger (Zimmern et al., P.N.A.S. 75: 4257-4261, 1978) dideoxy sequencing techniques were used to complete the determination of the nucleotide sequence. A continuous DNA sequence of 7,209 nucleotides was obtained using the Intelligenetics software package. This nucleotide sequence and the amino acids inferred therefrom follow.

TTAAAACAGCG GATGGGTATC CCACCATTCG ACCCATTGGG TGTAGTACTC
TGGTACTATG TACCTTTGTA CGCCTGTTTC TCCCCAACCA CCCTTCCTTA
AAATTCCCAC CCATGAAACG TTAGAAGCTT GACATTAAAG TACAATAGGT
GGCGCCATAT CCAATGGTGT CTATGTACAA GCACTTCTGT TTCCCAGGAG
CGAGGTATAG GCTGTACCCA CTGCCAAAAG CCTTTAACCG TTATCCGCCAA
CCAACTACGT AACAGTTAGT ACCATCTTGT TCTTGACTGG ACGTTCGATC
AGGTGGATTT TCCCTCCACT AGTTTGGTCG ATGAGGCTAG GAATTCCCCA
CGGGTGACCG TGTCCTAGCC TGCGTGGCGG CCAACCCAG CTTATGCTGG
GACGCCCTTT TAAGGACATG GTGTGAAGAC TCGCATGTGC TTGGTTGTGA
GTCCTCCGGC CCCTGAATGC GGCTAACCTT AACCCTAGAG CCTTATGCCA
CGATCCAGTG GTTGTAAGGT CGTAATGAGC AATTCCGGGA CGGGACCGAC
TACTTTGGGT GTCCGTGTTT CTCATTTTTC TTCATATTGT CTTATGGTCA
CAGCATATAT ATACATATAC TGTGATC

VP4

ATG GGC GCT CAG GTT TCT ACA CAG AAA AGT GGA TCT CAC
MET Gly Ala Gln Val Ser Thr Gln Lys Ser Gly Ser His

GAA AAT CAA AAC ATT TTG ACC AAT GGA TCA AAT CAG ACT
Glu Asn Gln Asn Ile Leu Thr Asn Gly Ser Asn Gln Thr

TTC ACA GTT ATA AAT TAC TAT AAG GAT GCA GCA AGT ACA
Phe Thr Val Ile Asn Tyr Tyr Lys Asp Ala Ala Ser Thr

TCA TCA GCT GGT CAA TCA CTG TCA ATG GAC CCA TCT AAG
Ser Ser Ala Gly Gln Ser Leu Ser MET Asp Pro Ser Lys

TTT ACA GAA CCA GTT AAA GAT CTC ATG CTT AAG GGT GCA
Phe Thr Glu Pro Val Lys Asp Leu MET Leu Lys Gly Ala

VP4 VP2

CCA GCA TTG ATT TCA CCC AAT GTT GAG GCC TGT GGT TAT
Pro Ala Leu Asn Ser Pro Asn Val Glu Ala Cys Gly Tyr

AGT GAT AGA GTA CAA CAA ATC ACA CTC GGG AAT TCA ACA
Ser Asp Arg Val Gln Gln Ile Thr Leu Gly Asn Ser Thr

ATA ACA ACA CAA GAA GCA GCC AAC GCT GTT GTG TGT TAT
Ile Thr Thr Gln Glu Ala Ala Asn Ala Val Val Cys Tyr

GCT GAA TGG CCA GAG TAC CTT CCA GAT GTG GAC GCT AGT
Ala Glu Trp Pro Glu Tyr Leu Pro Asp Val Asp Ala Ser

GAT GTC AAT AAA ACT TCA AAA CCA GAC ACT TCT GTC TGT
Asp Val Asn Lys Thr Ser Lys Pro Asp Thr Ser Val Cys

AGG TTT TAC ACA TTG GAT AGT AAG ACA TGG ACA ACA GGT
Arg Phe Tyr Thr Leu Asp Ser Lys Thr Trp Thr Thr Gly

TCT AAA GGC TGG TGC TGG AAA TTA CCA GAT GCA CTC AAG
Ser Lys Gly Trp Cys Trp Lys Leu Pro Asp Ala Leu Lys

GAT ATG GGT GTG TTC GGG CAA AAC ATG TTT TTC CAC TCA
Asp MET Gly Val Phe Gly Gln Asn MET Phe Phe His Ser

CTA GGA AGA TCA GGT TAC ACA GTA CAC GTT CAG TGC AAT
Leu Gly Arg Ser Gly Tyr Thr Val His Val Gln Cys Asn

GCC ACA AAA TTC CAT AGC GGT TGT CTA CTT GTA GTT GTA
Ala Thr Lys Phe His Ser Gly Cys Leu Leu Val Val Val

```
ATA CCA GAA CAC CAA CTG GCT TCA CAT GAG GGT GGC AAT
Ile Pro Glu His Gln Leu Ala Ser His Glu Gly Gly Asn

GTT TCA GTT AAA TAC ACA TTC ACG CAT CCA GGT GAA CGT
Val Ser Val Lys Tyr Thr Phe Thr His Pro Gly Glu Arg

GGT ATA GAT TTA TCA TCT GCA AAT GAA GTG GGA GGG CCT
Gly Ile Asp Leu Ser Ser Ala Asn Glu Val Gly Gly Pro

GTC AAG GAT GTC ATA TAC AAT ATG AAT GGT ACT TTA TTA
Val Lys Asp Val Ile Tyr Asn MET Asn Gly Thr Leu Leu

GGA AAT CTG CTC ATT TTC CCT CAC CAG TTC ATT AAT CTA
Gly Asn Leu Leu Ile Phe Pro His Gln Phe Ile Asn Leu

AGA ACC AAT AAT ACA GCC ACA ATA GTG ATA CCA TAC ATA
Arg Thr Asn Asn Thr Ala Thr Ile Val Ile Pro Tyr Ile

AAC TCA GTA CCC ATT GAT TCA ATG ACA CGT CAC AAC AAT
Asn Ser Val Pro Ile Asp Ser MET Thr Arg His Asn Asn

GTC TCA CTG ATG GTC ATC CCT ATT GCC CCT CTT ACA GTA
Val Ser Leu MET Val Ile Pro Ile Ala Pro Leu Thr Val

CCA ACT GGA GCA ACT CCC TCA CTC CCT ATA ACA GTC ACA
Pro Thr Gly Ala Thr Pro Ser Leu Pro Ile Thr Val Thr

ATA GCA CCT ATG TGC ACT GAG TTC TCT GGG ATA AGG TCC
Ile Ala Pro MET Cys Thr Glu Phe Ser Gly Ile Arg Ser
```

VP2 VP3

```
AAG TCA ATT GTG CCA CAA GGT TTG CCA ACT ACA ACT TTG
Lys Ser Ile Val Pro Gln Gly Leu Pro Thr Thr Thr Leu

CCG GGG TCA GGA CAA TTC TTG ACC ACA GAT GAC AGG CAA
Pro Gly Ser Gly Gln Phe Leu Thr Thr Asp Asp Arg Gln

TCC CCC AGT GCA CTG CCA AAT TAT GAG CCA ACT CCA AGA
Ser Pro Ser Ala Leu Pro Asn Tyr Glu Pro Thr Pro Arg

ATA CAC ATA CTA GGG AAA GTT CAT AAC TTG CTA GAA ATT
Ile His Ile Leu Gly Lys Val His Asn Leu Leu Glu Ile

ATA CAG GTA GAT ACA CTC ATT CCT ATG AAC AAC ACG CAT
Ile Gln Val Asp Thr Leu Ile Pro MET Asn Asn Thr His

ACA AAA GAT GAG GTT AAC AGT TAC CTC ATA CCA CTA AAT
Thr Lys Asp Glu Val Asn Ser Tyr Leu Ile Pro Leu Asn

GCA AAC AGG CAA AAT GAG CAG GTT TTT GGG ACA AAC CTG
Ala Asn Arg Gln Asn Glu Gln Val Phe Gly Thr Asn Leu

TTT ATT GGT GAT GGG GTC TTC AAA ACT ACT CTT CTG GGT
Phe Ile Gly Asp Gly Val Phe Lys Thr Thr Leu Leu Gly

GAA ATT GTT CAG TAC TAT ACA CAT TGG TCT GGA TCA CTT
Glu Ile Val Gln Tyr Tyr Thr His Trp Ser Gly Ser Leu

AGA TTC TCT TCG ATG TAT ACT GGT CCT GCC TTG TCC AGT
Arg Phe Ser Ser MET Tyr Thr Gly Pro Ala Leu Ser Ser
```

GCT AAA CTC ACT CTA GCA TAC ACC CCG CCT GGT GCT CGT
Ala Lys Leu Thr Leu Ala Tyr Thr Pro Pro Gly Ala Arg

GGT CCA CAG GAC AGG AGA GAA GCA ATG CTA GGT ACT CAT
Gly Pro Gln Asp Arg Arg Glu Ala MET Leu Gly Thr His

GTT GTC TGG GAT ATT GGT CTG CAA TCC ACC ATA GTA ATG
Val Val Trp Asp Ile Gly Leu Gln Ser Thr Ile Val MET

ACA ATA CCA TGG ACA TCA GGG GTG CAG TTT AGA TAT ACT
Thr Ile Pro Trp Thr Ser Gly Val Gln Phe Arg Tyr Thr

GAT CCA GAT ACA TAC ACC AGT GCT GGC TTT CTA TCA TGT
Asp Pro Asp Thr Tyr Thr Ser Ala Gly Phe Leu Ser Cys

TGG TAT CAA ACT TCT CTT ATA CTT CCC CCA GAA ACG ACC
Trp Tyr Gln Thr Ser Leu Ile Leu Pro Pro Glu Thr Thr

GGC CAG GTC TAC TTA TTA TCA TTC ATA AGT GCA TGT CCA
Gly Gln Val Tyr Leu Leu Ser Phe Ile Ser Ala Cys Pro

GAT TTT AAG CTT AGG CTG ATG AAA GAT ACT CAA ACT ATC
Asp Phe Lys Leu Arg Leu MET Lys Asp Thr Gln Thr Ile

VP3 VP1

TCA CAG ACT GTT GCA CTC ACT GAA GGC TTA GGT GAT GAA
Ser Gln Thr Val Ala Leu Thr Glu Gly Leu Gly Asp Glu

TTA GAA GAA GTC ATC GTT GAG AAA ACG AAA CAG ACG GTG
Leu Glu Glu Val Ile Val Glu Lys Thr Lys Gln Thr Val

```
GCC TCA ATC TCA TCT GGT CCA AAA CAC ACA CAA AAA GTC
Ala Ser Ile Ser Ser Gly Pro Lys His Thr Gln Lys Val


CCC ATA CTA ACT GCA AAC GAA ACA GGG GCC ACA ATG CCT
Pro Ile Leu Thr Ala Asn Glu Thr Gly Ala Thr MET Pro


GTT CTT CCA TCA GAC AGC ATA GAA ACC AGA ACT ACC TAC
Val Leu Pro Ser Asp Ser Ile Glu Thr Arg Thr Thr Tyr


ATG CAC TTT AAT GGT TCA GAA ACT GAT GTA GAA TGC TTT
MET His Phe Asn Gly Ser Glu Thr Asp Val Glu Cys Phe


TTG GGT CGT GCA GCT TGT GTG CAT GTA ACT GAA ATA CAA
Leu Gly Arg Ala Ala Cys Val His Val Thr Glu Ile Gln


AAC AAA GAT GCT ACT GGA ATA GAT AAT CAC AGA GAA GCA
Asn Lys Asp Ala Thr Gly Ile Asp Asn His Arg Glu Ala


AAA TTG TTC AAT GAT TGG AAA ATC AAC CTG TCC AGC CTT
Lys Leu Phe Asn Asp Trp Lys Ile Asn Leu Ser Ser Leu


GTC CAA CTT AGA AAG AAA CTG GAA CTC TTC ACT TAT GTT
Val Gln Leu Arg Lys Lys Leu Glu Leu Phe Thr Tyr Val


AGG TTT GAT TCT GAG TAT ACC ATA CTG GCC ACT GCA TCT
Arg Phe Asp Ser Glu Tyr Thr Ile Leu Ala Thr Ala Ser


CAA CCT GAT TCA GCA AAC TAT TCA AGC AAT TTG GTG GTC
Gln Pro Asp Ser Ala Asn Tyr Ser Ser Asn Leu Val Val
```

```
CAA GCC ATG TAT GTT CCA CAT GGT GCC CCG AAA TCC AAA
Gln Ala MET Tyr Val Pro His Gly Ala Pro Lys Ser Lys


AGA GTG GGC GAT TAC ACA TGG CAA AGT GCT TCA AAC CCC
Arg Val Gly Asp Tyr Thr Trp Gln Ser Ala Ser Asn Pro


AGT GTA TTC TTC AAG GTG GGG GAT ACA TCA AGG TTT AGT
Ser Val Phe Phe Lys Val Gly Asp Thr Ser Arg Phe Ser


GTG CCT TAT GTA GGA TTG GCA TCA GCA TAT AAT TGT TTT
Val Pro Tyr Val Gly Leu Ala Ser Ala Tyr Asn Cys Phe


TAT GAT GGT TAC TCA CAT GAT GAT GCA GAA ACT CAG TAT
Tyr Asp Gly Tyr Ser His Asp Asp Ala Glu Thr Gln Tyr


GGC ATA ACT GTT CTA AAC CAT ATG GGT AGT ATG GCA TTC
Gly Ile Thr Val Leu Asn His MET Gly Ser MET Ala Phe


AGA ATA GTA AAT GAA CAT GAT GAA CAC AAA ACT CTT GTC
Arg Ile Val Asn Glu His Asp Glu His Lys Thr Leu Val


AAG ATC AGA GTT TAT CAC AGG GCA AAG CTC GTT GAA GCA
Lys Ile Arg Val Tyr His Arg Ala Lys Leu Val Glu Ala


TGG ATT CCA AGA GCA CCC AGA GCA CTA CCC TAC ACA TCA
Trp Ile Pro Arg Ala Pro Arg Ala Leu Pro Tyr Thr Ser


ATA GGG CGC ACA AAT TAT CCT AAG AAT ACA GAA CCA GTA
Ile Gly Arg Thr Asn Tyr Pro Lys Asn Thr Glu Pro Val
```

VP1 3B

ATT AAG AAG AGG AAA GGT GAC ATT AAA TCC TAT GGT TTA
Ile Lys Lys Arg Lys Gly Asp Ile Lys Ser Tyr Gly Leu

GGA CCT AGG TAC GGT GGG ATT TAT ACA TCA AAT GTT AAA
Gly Pro Arg Tyr Gly Gly Ile Tyr Thr Ser Asn Val Lys

ATA ATG AAT TAC CAC TTG ATG ACA CCA GAA GAC CAC CAT
Ile MET Asn Tyr His Leu MET Thr Pro Glu Asp His His

AAT CTG ATA GCA CCC TAT CCA AAT AGA GAT TTA GCA ATA
Asn Leu Ile Ala Pro Tyr Pro Asn Arg Asp Leu Ala Ile

GTC TCA ACA GGA GGA CAT GGT GCA GAA ACA ATA CCA CAC
Val Ser Thr Gly Gly His Gly Ala Glu Thr Ile Pro His

TGT AAC CGT ACA TCA GGT GTT TAC TAT TCC ACA TAT TAC
Cys Asn Arg Thr Ser Gly Val Tyr Tyr Ser Thr Tyr Tyr

AGA AAG TAT TAC CCC ATA ATT TGC GAA AAG CCC ACC AAC ATC
Arg Lys Tyr Tyr Pro Ile Ile Cys Glu Lys Pro Thr Asn Ile

TGG ATT GAA GGA AGC CCT TAT TAC CCA AGT AGA TTT CAA
Trp Ile Glu Gly Ser Pro Tyr Tyr Pro Ser Arg Phe Gln

GCA GGA GTG ATG AAA GGG GTT GGG CCA GCA GAG CTA GGA
Ala Gly Val MET Lys Gly Val Gly Pro Ala Glu Leu Gly

GAC TGC GGT GGG ATT TTG AGA TGC ATA CAT GGT CCC ATT
Asp Cys Gly Gly Ile Leu Arg Cys Ile His Gly Pro Ile

GGA TTG TTA ACA GCT GAA GGT AGT GGA TAT GTT TGT TTT
Gly Leu Leu Thr Ala Glu Gly Ser Gly Tyr Val Cys Phe

3B

GCT GAC ATA CGA CAG TTG GAG TGT ATC GCA GAG GAA CAG
Ala Asp Ile Arg Gln Leu Glu Cys Ile Ala Glu Glu Gln

5B

GGG CTG AGT GAT TAC ATC ACA GGT TTG GGT AGA GCT TTT
Gly Leu Ser Asp Tyr Ile Thr Gly Leu Gly Arg Ala Phe

GGT GTC GGG TTC ACT GAC CAA ATC TCA ACA AAA GTC ACA
Gly Val Gly Phe Thr Asp Gln Ile Ser Thr Lys Val Thr

GAA CTA CAA GAA GTG GCG AAA GAT TTC CTC ACC ACA AAA
Glu Leu Gln Glu Val Ala Lys Asp Phe Leu Thr Thr Lys

GTT TTG TCC AAA GTG GTC AAA ATG GTT TCA GCT TTA GTG
Val Leu Ser Lys Val Val Lys MET Val Ser Ala Leu Val

ATC ATT TGC AGA AAT CAT GAT GAC TTG GTC ACT GTT ACG
Ile Ile Cys Arg Asn His Asp Asp Leu Val Thr Val Thr

GCC ACT CTA GCA CTA CTT GGA TGT GAT GGA TCT CCT TGG
Ala Thr Leu Ala Leu Leu Gly Cys Asp Gly Ser Pro Trp

AGA TTT CTG AAG ATG TAC ATT TCC AAA CAC TTT CAG GTG
Arg Phe Leu Lys MET Tyr Ile Ser Lys His Phe Gln Val

5B X

CCT TAC ATT GAA AGA CAA GCA AAT GAT GGA TGG TTC AGA
Pro Tyr Ile Glu Arg Gln Ala Asn Asp Gly Trp Phe Arg

```
      AAG TTT AAT GAT GCA TGT AAT GCT GCA AAG GGA TTG GAA
      Lys Phe Asn Asp Ala Cys Asn Ala Ala Lys Gly Leu Glu


      TGG ATT GCT AAT AAG ATT TCC AAA CTG ATT GAA TGG ATA
      Trp Ile Ala Asn Lys Ile Ser Lys Leu Ile Glu Trp Ile


      AAA AAC AAA GTA CTT CCC CAA GCC AAA GAA AAA CTA GAA
      Lys Asn Lys Val Leu Pro Gln Ala Lys Glu Lys Leu Glu


      TTT TGT AGT AAA CTC AAA CAA CTT GAT ATA CTA GAG AGA
      Phe Cys Ser Lys Leu Lys Gln Leu Asp Ile Leu Glu Arg


      CAA ATA ACC ACC ATG CAT ATC TCG AAT CCA ACA CAG GAA
      Gln Ile Thr Thr MET His Ile Ser Asn Pro Thr Gln Glu


      AAA CGA GAG CAG TTG TTC AAT AAC GTA TTG TGG TTG GAA
      Lys Arg Glu Gln Leu Phe Asn Asn Val Leu Trp Leu Glu


      CAA ATG TCG CAA AAG TTT GCC CCA TTT TAT GCC GTT GAA
      Gln MET Ser Gln Lys Phe Ala Pro Phe Tyr Ala Val Glu


      TCA AAA AGA ATC AGG GAA CTC AAG AAC AAA ATG GTA AAT
      Ser Lys Arg Ile Arg Glu Leu Lys Asn Lys MET Val Asn


      TAT ATG CAA TTT AAA AGT AAA CAA AGA ACT GAA CCA GTG
      Tyr MET Gln Phe Lys Ser Lys Gln Arg Thr Glu Pro Val


      TGT GTA TTA ATC CAT GGT ACA CCC GGT TCT GGT AAA TCA
      Cys Val Leu Ile His Gly Thr Pro Gly Ser Gly Lys Ser


      TTA ACA ACA TCC ATT GTG GGA CGT GCA ATT GCA GAA CAC
      Leu Thr Thr Ser Ile Val Gly Arg Ala Ile Ala Glu His
```

TTC AAT TCA GCA GTA TAT TCA CTT CCA CCA GAT CCC AAG
Phe Asn Ser Ala Val Tyr Ser Leu Pro Pro Asp Pro Lys

CAC TTT GAT GGT TAT CAG CAA CAG GAA GTT GTG ATT ATG
His Phe Asp Gly Tyr Gln Gln Gln Glu Val Val Ile MET

GAT GAT CTG AAC CAA AAT CCA GAT GGA CAG GAT ATA AGC
Asp Asp Leu Asn Gln Asn Pro Asp Gly Gln Asp Ile Ser

ATG TTT TGT CAA ATG GTT TCT TCA GTG GAT TTC TTG CCT
MET Phe Cys Gln MET Val Ser Ser Val Asp Phe Leu Pro

CCA ATG GCT AGT TTA GAT AAC AAG GGC ATG TTA TTC ACC
Pro MET Ala Ser Leu Asp Asn Lys Gly MET Leu Phe Thr

AGT AAT TTT GTT CTA GCC TCC ACA AAT TCT AAC ACA CTA
Ser Asn Phe Val Leu Ala Ser Thr Asn Ser Asn Thr Leu

AGC CCC CCA ACA ATC TTG AAT CCT GAA GCT TTA GTC AGG
Ser Pro Pro Thr Ile Leu Asn Pro Glu Ala Leu Val Arg

AGA TTT GGT TTT GAC CTA GAT ATA TGT TTG CAT ACT ACC
Arg Phe Gly Phe Asp Leu Asp Ile Cys Leu His Thr Thr

TAC ACA AAG AAT GGA AAA CTC AAT GCA GGC ATG TCA ACC
Tyr Thr Lys Asn Gly Lys Leu Asn Ala Gly MET Ser Thr

AAG ACA TGC AAA GAT TGC CAT CAA CCA TCT AAT TTC AAG
Lys Thr Cys Lys Asp Cys His Gln Pro Ser Asn Phe Lys

AAA TGT TGC CCC CTA GTC TGT GGA AAA GCT ATT AGC TTG
Lys Cys Cys Pro Leu Val Cys Gly Lys Ala Ile Ser Leu

GTA GAC AGA ACT ACC AAC GTT AGG TAT AGT GTG GAT CAA
Val Asp Arg Thr Thr Asn Val Arg Tyr Ser Val Asp Gln

CTG GTC ACG GCT ATT ATA AGT GAT TTC AAG AGC AAA ATG
Leu Val Thr Ala Ile Ile Ser Asp Phe Lys Ser Lys MET

X 1B

CAA ATT ACA GAT TCC CTA GAA ACA CTG TTT CAA GGA CCA
Gln Ile Thr Asp Ser Leu Glu Thr Leu Phe Gln Gly Pro

GTG TAT AAA GAT TTA GAG ATT GAT GTT TGC AAC ACA CCA
Val Tyr Lys Asp Leu Glu Ile Asp Val Cys Asn Thr Pro

CCT TCA GAA TGT ATC AAC GAT TTA CTG AAA TCT GTA GAT
Pro Ser Glu Cys Ile Asn Asp Leu Leu Lys Ser Val Asp

TCA GAA GAG ATT AGG GAA TAT TGT AAG AAG AAG AAA TGG
Ser Glu Glu Ile Arg Glu Tyr Cys Lys Lys Lys Lys Trp

ATT ATA CCT GAA ATT CCT ACC AAC ATA GAA AGG GCT ATG
Ile Ile Pro Glu Ile Pro Thr Asn Ile Glu Arg Ala MET

AAT CAA GCC AGC ATG ATT ATT AAT ACT ATT CTG ATG TTT
Asn Gln Ala Ser Met Ile Ile Asn Thr Ile Leu MET Phe

```
         GTC AGT ACA TTA GGT ATT GTT TAT GTC ATT TAT AAA TTG
         Val Ser Thr Leu Gly Ile Val Tyr Val Ile Tyr Lys Leu


                       1B VPg
         TTT GCT CAA ACT CAA GGA CCA TAT TCT GGT AAC CCG CCT
         Phe Ala Gln Thr Gln Gly Pro Tyr Ser Gly Asn Pro Pro


         CAC AAT AAA CTA AAA GCC CCA ACT TTA CGC CCA GTT GTT
         His Asn Lys Leu Lys Ala Pro Thr Leu Arg Pro Val Val


             VPg Protease
         GTG CAA GGA CCA AAC ACA GAA TTT GCA CTA TCC CTG TTA
         Val Gln Gly Pro Asn Thr Glu Phe Ala Leu Ser Leu Leu


         AGG AAA AAC ATA ATG ACT ATA ACA ACC TCA AAG GGA GAG
         Arg Lys Asn Ile MET Thr Ile Thr Thr Ser Lys Gly Glu


         TTC ACA GGG TTA GGC ATA CAT GAT CGT GTC TGT GTG ATA
         Phe Thr Gly Leu Gly Ile His Asp Arg Val Cys Val Ile


         CCC ACA CAC GCA CAG CCT GGT GAT GAT GTA CTA GTG AAT
         Pro Thr His Ala Gln Pro Gly Asp Asp Val Leu Val Asn


         GGT CAG AAA ATT AGA GTT AAG GAT AAG TAC AAA TTA GTA
         Gly Gln Lys Ile Arg Val Lys Asp Lys Tyr Lys Leu Val


         GAT CCA GAG AAC ATT AAT CTA GAG CTT ACA GTG TTG ACT
         Asp Pro Glu Asn Ile Asn Leu Glu Leu Thr Val Leu Thr


         TTA GAT AGA AAT GAA AAA TTC AGA GAT ATC AGG GGA TTT
         Leu Asp Arg Asn Glu Lys Phe Arg Asp Ile Arg Gly Phe
```

ATA TCA GAA GAT CTA GAA GGT GTG GAT GCC ACT TTG GTA
Ile Ser Glu Asp Leu Glu Gly Val Asp Ala Thr Leu Val

GTA CAT TCA AAT AAC TTT ACC AAC ACT ATC TTA GAA GTT
Val His Ser Asn Asn Phe Thr Asn Thr Ile Leu Glu Val

GGC CCT GTA ACA ATG GCA GGA CTT ATT AAT TTG AGT AGC
Gly Pro Val Thr MET Ala Gly Leu Ile Asn Leu Ser Ser

ACC CCC ACT AAC AGA ATG ATT CGT TAT GAT TAT GCA ACA
Thr Pro Thr Asn Arg MET Ile Arg Tyr Asp Tyr Ala Thr

AAA ACT GGG CAG TGT GGA GGT GTG CTG TGT GCT ACT GGT
Lys Thr Gly Gln Cys Gly Gly Val Leu Cys Ala Thr Gly

AAG ATC TTT GGT ATT CAT GTT GGC GGT AAT GGA AGA CAA
Lys Ile Phe Gly Ile His Val Gly Gly Asn Gly Arg Gln

GGA TTT TCA GCT CAA CTï AAA AAA CAA TAT TTT GTA GAG
Gly Phe Ser Ala Gln Leu Lys Lys Gln Tyr Phe Val Glu

Protease Replicase
AAA CAA   GGC CAA GTA ATA GCT AGA CAT AAG GTT AGG GAG
Lys Gln   Gly Gln Val Ile Ala Arg His Lys Val Arg Glu

TTT AAC ATA AAT CCA GTC AAC ACG GCA ACT AAG TCA AAA
Phe Asn Ile Asn Pro Val Asn Thr Ala Thr Lys Ser Lys

TTA CAT CCC AGT GTA TTT TAT GAT GTT TTT CCA GGT GAC
Leu His Pro Ser Val Phe Tyr Asp Val Phe Pro Gly Asp

```
AAG GAA CCT GCT GTA TTG AGT GAC AAT GAT CCC AGA CTG
Lys Glu Pro Ala Val Leu Ser Asp Asn Asp Pro Arg Leu

GAA GTT AAA TTG ACT GAA TCA TTA TTC TCT AAG TAC AAG
Glu Val Lys Leu Thr Glu Ser Leu Phe Ser Lys Tyr Lys

GGG AAT GTA AAT ACG GAA CCC ACT GAA AAT ATG CTT GTG
Gly Asn Val Asn Thr Glu Pro Thr Glu Asn MET Leu Val

GCT GTA GAC CAT TAT GCA GGG CAA CTA TTA TCA CTA GAT
Ala Val Asp His Tyr Ala Gly Gln Leu Leu Ser Leu Asp

ATC CCC ACT TCT GAA CTT ACA CTA AAA GAA GCA TTA TAT
Ile Pro Thr Ser Glu Leu Thr Leu Lys Glu Ala Leu Tyr

GGA GTA GAT GGA CTA GAA CCT ATA GAT ATT ACA ACC AGT
Gly Val Asp Gly Leu Glu Pro Ile Asp Ile Thr Thr Ser

GCA GGA TTT CCC TAT GTG AGT CTT GGG ATC AAA AAG AGA
Ala Gly Phe Pro Tyr Val Ser Leu Gly Ile Lys Lys Arg

GAC ATT CTG AAT AAA GAG ACC CAG GAC ACA GAA AAG ATG
Asp Ile Leu Asn Lys Glu Thr Gln Asp Thr Glu Lys MET

AAG TTT TAT CTA GAC AAG TAT GGC ATT GAC TTG CCT CTA
Lys Phe Tyr Leu Asp Lys Tyr Gly Ile Asp Leu Pro Leu

GTT ACA TAT ATT AAG GAT GAA TTA AGA AGT GTT GAC AAA
Val Thr Tyr Ile Lys Asp Glu Leu Arg Ser Val Asp Lys
```

```
GTC CGA TTA GGG AAA AGT AGA TTA ATT GAA GCC TCC AGT
Val Arg Leu Gly Lys Ser Arg Leu Ile Glu Ala Ser Ser


TTG AAT GAT TCT GTT AAC ATG AGA ATG AAA CTA GGC AAC
Leu Asn Asp Ser Val Asn MET Arg MET Lys Leu Gly Asn


CTT TAC AAA GCA TTC CAT CAA AAT CCC GGT GTT CTG ACT
Leu Tyr Lys Ala Phe His Gln Asn Pro Gly Val Leu Thr


GGA TCA GCA GTG GGT TGT GAT CCT GAT GTG TTT TGG TCT
Gly Ser Ala Val Gly Cys Asp Pro Asp Val Phe Trp Ser


GTC ATC CCT TGC TTA ATG GAT GGG CAC CTG ATG GCA TTT
Val Ile Pro Cys Leu MET Asp Gly His Leu MET Ala Phe


GAT TAC TCT AAT TTT GAT GCC TCT TTG TCA CCA GTT TGG
Asp Tyr Ser Asn Phe Asp Ala Ser Leu Ser Pro Val Trp


TTT GTC TGT CTA GAG AAG GTT TTG ACC AAG TTA GGC TTT
Phe Val Cys Leu Glu Lys Val Leu Thr Lys Leu Gly Phe


GCA GGC TCT TCA TTA ATT CAA TCA ATT TGT AAT ACC CAT
Ala Gly Ser Ser Leu Ile Gln Ser Ile Cys Asn Thr His


CAT ATC TTT AGG GAT GAA ATA TAT GTG GTT GAA GGT GGC
His Ile Phe Arg Asp Glu Ile Tyr Val Val Glu Gly Gly


ATG CCC TCA GGG TGT TCA GGA ACC AGC ATA TTC AAT TCC
MET Pro Ser Gly Cys Ser Gly Thr Ser Ile Phe Asn Ser


ATG ATC AAC AAC ATA ATC ATT AGG ACT TTG ATA TTA GAT
MET Ile Asn Asn Ile Ile Ile Arg Thr Leu Ile Leu Asp
```

```
GCA TAT AAA GGA ATA GAT TTA GAC AAA CTT AAA ATC TTA
Ala Tyr Lys Gly Ile Asp Leu Asp Lys Leu Lys Ile Leu

GCT TAC GGT GAT GAT TTG ATT GTT TCT TAT CCT TAT GAA
Ala Tyr Gly Asp Asp Leu Ile Val Ser Tyr Pro Tyr Glu
CTG GAT CCA CAA GTG TTG GCA ACT CTT GGT AAA AAT TAT
Leu Asp Pro Gln Val Leu Ala Thr Leu Gly Lys Asn Tyr

GGA CTA ACC ATC ACA CCC CCA GAC AAA TCT GAA ACT TTT
Gly Leu Thr Ile Thr Pro Pro Asp Lys Ser Glu Thr Phe

ACA AAA ATG ACA TGG GAA AAC TTG ACA TTT TTA AAG AGA
Thr Lys MET Thr Trp Glu Asn Leu Thr Phe Leu Lys Arg

TAC TTC AAG CCT GAT CAA CAA TTT CCC TTT TTG GTT CAC
Tyr Phe Lys Pro Asp Gln Gln Phe Pro Phe Leu Val His

CCA GTT ATG CCC ATG AAA GAT ATA CAT GAG TCA ATC AGA
Pro Val MET Pro MET Lys Asp Ile His Glu Ser Ile Arg

TGG ACA AAG GAT CCT AAA AAC ACA CAG GAT CAC GTC CGA
Trp Thr Lys Asp Pro Lys Asn Thr Gln Asp His Val Arg

TCA TTA TGC ATG TTA GCA TGG CAC TCA GGA GAA AAA GAG
Ser Leu Cys MET Leu Ala Trp His Ser Gly Glu Lys Glu

TAC AAT GAA TTC ATT CAG AAG ATC AGA ACT ACT GAC ATT
Tyr Asn Glu Phe Ile Gln Lys Ile Arg Thr Thr Asp Ile

GGA AAA TGT CTA ATT CTC CCA GAA TAC AGC GTA CTT AGG
Gly Lys Cys Leu Ile Leu Pro Glu Tyr Ser Val Leu Arg
```

Replicase

AGG CGC TGG TTG GAC CTC TTT
Arg Arg Trp Leu Asp Leu Phe


TAGGTTAACAATATAGACACTTAATTTGAGTAGAAGTAGGAGT


TTATAAAAAA AAAAAAAAA


Nucleotide and corresponding amino acid sequences for the various structural and non-structural HRV14 proteins are indicated. Nomenclature employed is based upon that used for the polioviruses. Neutralizing epitopes to HRV14 have been mapped to each of the VP1, VP2 and VP3 virus structural proteins. The virus protease is required for proper proteolytic cleavage of the polyprotein; inhibition of its activity (e.g. by $Zn^{++}$) results in blockage of virus replication. Virus coded replicase and VP-g is required for synthesis of both plus and minus strands of genomic RNA; inhibition of its activity prevents virus replication.


### EXAMPLE 11

### Immunization of Mice with Purified Rhinovirus Type 14

The rear footpads of adult BALB/C mice were injected with purified whole HRV-14 as obtained according to Example 1. Each mouse footpad received 5 µg of purified HRV-14 dissolved in a volume of 0.1 ml of complete Freund's adjuvant. Mice received a second inoculation (5 µg/0.05 ml/footpad) at 30 days. Serum was collected from one eye of each mouse 14 days after viral injection and tested for antibody to HRV-14 by neutralization assay (Example 14).

Mice that were positive for anti-HRV-14 antibodies by neutralization assays (as described in Example 14) were primed 3 days prior to the cell fusion by injection of 5 µg of purified HRV-14 in the tail vein (in aqueous solution, 0.1 ml/mouse). Sera collected at death from the two animals used for the cell fusion were assayed for antibody to HRV-14 by neutralization and found to be positive.


## EXAMPLE 12
### Immunization of Mice with HeLa R19 Cells

Adult BALB/C mice were injected intraperitoneally with a solution containing HeLa R19 cells (Example 1) which were recovered from tissue culture monolayers by treatment with phosphate buffered saline (PBS) containing 50 mM EDTA for 20 mins. at 37°C. Each mouse received $3 \times 10^6$ cells suspended in a volume of 0.5 ml of complete Freund's adjuvant. After 38 days, mice were reinoculated intraperitoneally with $1 \times 10^7$ HeLa R19 cells in a volume of 0.5 ml of incomplete Freund's adjuvant.

Three mice that were positive for antibodies which protected cells against HRV-14 infection (See Example 14) were primed on day 116, 4 days prior to the cell fusion by injection of two $OD_{260}$ units/mouse of crude HeLa cell membranes (See Example 13) in the tail vein (in aqueous solution, 0.05 ml mouse). Sera collected at the death of the three animals used showed the presence of antibody capable of protecting HeLa cells from HRV-14 infection.

In another experiment three adult BALB/C mice were immunized using the following schedule: 2 intraperitoneal inoculations using cell membrane preparations on day 1 and day 42 followed by a final intradermal inoculation of membranes plus incomplete Freund's adjuvant on day 214. Three days later the cell fusion was carried out as described in Example 14.

### EXAMPLE 13

#### Preparation of HeLa Cell Membranes

HeLa R19 cell monolayers were treated with PBS containing 50 mM EDTA for 20 minutes at 37°C. Cells were collected by low speed centrifugation and washed with PBS 3 times. Cells were pelleted and resuspended in 10 mM phosphate buffer ($8 \times 10^{7}$ cells/ml) and placed on ice for 25 mins. Cells were then disrupted by 20 stokes in a Dounce homogenizer and nuclei removed by centrifugation (1000 x g for 5 minutes at 4°C). The supernatant liquid was removed and membranes pelleted at 143,000 x g for 1 hour at 4°C in a Beckman TI60 rotor. The supernatant liquid was discarded and the crude membrane pellet dissolved in PBS at 42 $OD_{280}$ units/ml and stored at -70°C.

### EXAMPLE 14

#### Production of Hybridomas by Fusion of Immune Mouse Spleen Cells with Mouse Myeloma Cells

Mouse myeloma cells (SP 2/0) were grown from frozen seed stock in HT media. Each 100 ml of HT media contained 66 ml of Dulbecco's Modified Eagle's Media; 20 ml fetal calf serum (FCS); 10 ml of NCTC 109 with Eagle's balanced salts; 2 ml of 200 mM L-glutamine; 1 ml of a solution containing 408 mg hypoxanthine and 114 mg thymidine in 300 ml of distilled $H_2O$; and 1 ml of OPI stock (1.5 g cis-oxalacetic acid, 0.5 g pyruvic acid, 2000 units bovine insulin in 100 ml $H_2O$); and 0.2 ml of a penicillin (10,000 units/ml) and streptomycin (10,000 µg/ml) mixture. The mouse spleens from animals immunized with HRV-14 (Example 12) were removed after cervical dislocation and placed in serumless HT media at room temperature. The spleens were placed in a plastic petri dish and the cells were teased from the spleen with a plastic scraper. The plates were washed with 5 ml of serumless HT media and the cells were pooled into a 15 ml plastic conical tube; large particles were allowed to settle for one minute. The supernatant was then transferred to a 15 ml plastic round bottom tube and the cells were pelleted by a 10 minute centrifugation, 350 x g at room temperature. The supernatant liquid was discarded and the cells were resuspended in serumless HT media (10 ml/2 spleens) and the total viable cells were determined by trypan blue exclusion.

The number of viable SP 2/0 myeloma cells was also determined and the cells were combined using one log more spleen cells than myeloma cells (i.e. into a 50 cc screw cap plastic tube were placed $2 \times 10^8$ spleen cells and $2 \times 10^7$ SP 2/0 cells). The cells were pelleted by centrifugation for 10 minutes at 350 x g and then the cell pellet was resuspended in 10 ml of serumless HT media. This pelleting and resuspension-washing procedure was repeated two more times, and after the final resuspension in 10 ml of serumless HT media the cells were transferred to a 15 ml round bottom tube. The cells were pelleted at 350 x g for 10 minutes.

Polyethylene glycol (PEG; molecular weight average = 1000d) was liquified at 45°C and combined with serumless HT media to a concentration of 35% PEG (vol/vol). The PEG/HT media mixture was sterilized by passing it through a 0.22 micron membrane filter fitted on the end of 3 ml syringe; the PEG was then maintained at 37°C. Dimethylsulfoxide (DMSO) was added to the PEG/media mixture to a final concentration of 5%. The PEG/DMSO/HT media mixture was added dropwise to the spleen-myeloma cell pellett, using 0.8 ml for $2 \times 10^8$ cells while gently resuspending the cells by tapping the side of the culture tube and swirling the cells. The cell pellets were centrifuged at 250 x g at room temperature so that the total contact time of the cells with PEG was 6 minutes. The PEG supernatant was then aspirated off and the cells were resuspended in 10 ml of HT media. The cells were pelleted (350 x g for 10 minutes) and gently resuspended in HT media to a final concentration of $3.5 \times 10^5$ myeloma cells/ml. The cells were then plated in 96 well microtiter plates using 0.1 ml/well with a pipet and incubated at 37°C in a water-jacketed $CO_2$ incubator with 5% $CO_2$ and 96% humidity.

After 24 hours, 0.1 ml of HAT media (HT media plus aminopterin at 0.352 mg/liter) was added to each well. The wells were refed with 0.1 ml of fresh HAT media every 4 to 5 days.

Some cells from culture wells which were at a confluency of 15% or greater (usually 40 to 80%) were tested for production of antibodies to HRV-14 or by a neutralization assay described in Example 15.

In other experiments some cells from growth-positive wells which were at a confluency of 15% or greater were tested for production of antibodies which block attachment of HRV-14 to cellular receptors in a cell protection assay described in Example 15.

From the first set of three mice in Example 12, 837 growth-positive wells were obtained, and from the second set of three mice in Example 12, 693 growth-positive wells were obtained. Screening of culture fluids from the total 1530 wells resulted in the identification of two hybridoma cultures, one from each set of three mice, producing antibodies capable of blocking the attachment of the major group of human rhinoviruses to susceptible cells.

Those cells having high activity by the above assays were subcloned by two cycles of limiting dilutions. Eight subclones (A-H) were selected which had high HRV-14 neutralization activities and one subclone (I) which was selected in a cell protection assay.  The purified monoclonal antibodies from these subclones were characterized as to immunoglobulin subtype and molecular weight by standard procedure. The following results were obtained:

| MONOCLONAL ANTIBODY | SUBTYPE | MOLECULAR WEIGHT (Daltons) | |
|---|---|---|---|
| | | Heavy Chain | Light Chain |
| A | IgG-2A | 49,000 | 27,000 |
| B | IgG-2A | 49,000 | 27,000 |
| C | IgG-2A | 49,000 | 27,000 |
| D | IgG-2A | 49,000 | 27,000 |
| E | IgG-1 | 49,000 | 27,000 |
| F | IgG-2A | 49,000 | 27,000 |
| G | IgG-2A | 49,000 | 27,000 |
| H | IgG-2A | 49,000 | 27,000 |
| I | IgG-1 | 49,000 | 27,000 |

EXAMPLE 15

Neutralization/Cell Protection Assays

Neutralization assays were performed as follows:  HRV-14 ($10^6$ PFU) was incubated with 40 µl of serum or hydridoma tissue culture fluid in a total volume of 0.2 ml GM for 1 hour at 25°C.  The solution was then transferred to a HeLa R19 cell monolayer (2.5 $10^5$ cells) in a 24-well cluster plate and incubated 16-24 hours at 34°C in a $CO_2$ incubator. Wells were scored as positive if no evidence of cytopathic effect was evident compared to controls.

Protection assays utilized 48-well cluster plates of HeLa R-19 cells (1.25 x $10^5$ cells/well).

After removal of media, monolayers were incubated with 0.1 ml serum or hybridoma tissue culture for 1 hour at 34°C. After incubation, 2 x $10^5$ PFUs of HRV-14 in 50 µl of media were added to the treated monolayer and the cells incubated 16-24 hours at 34°C in a $CO_2$ incubator. Wells were scored as positive if no evidence of cytopathic effect was evident compared to controls. Further assays were done to determine if this HeLa receptor antibody would protect cells against infection with other serotypes of HRVs. Positive protection results were obtained with HRV types 3, 5, 9, 11, 12, 14, 15, 17, 22, 32, 36, 39, 41, 51, 58, 59, 60, 66, 67, 89, and a clinical isolate of unknown serotype, and coxsackie virus types A13, A18 and A21. All of these serotypes are known to share the same cellular receptor. Four serotypes of rhinovirus which share a different receptor (Types 1A, 2, 44, and 49) were not blocked by the antibody. This monoclonal antibody also failed to protect cells against Sabin Type 1 polio virus and coxsackie viruses Types B2 and B3.

## EXAMPLE 16
### Purification of Monoclonal antibodies from Tissue Culture Media and Ascites Fluid

The monoclonal antibodies used in experiments were purified from tissue culture fluid taken from growing cultures of the hybridoma cells producing individual antibody. The following procedure was employed and is essentially that described by Emini et al. (J. Virol. 43:997-1005, 1982). Tissue culture fluid, 1 liter, from a particular hybridoma cell line was filtered through a Whatmann 1MM filter paper, a glass wool containing column, and finally a column containing agarose (Sepharose 6B). The tissue culture fluid was then adjusted to a pH of 8.0 by the addition of 100 mM tris-HCl, pH 8.0, and then passed over a protein A-Sepharose column (4 to 6 ml bed volume per 2 liters of fluid). The column was washed with 10 bed volumes of 10 mM Tris-HCl (pH 8.0). Glycine (100 mM) pH 3.0 was then added to elute the bound immunoglobulins from the column; fractions were collected with 100 mM Tris-HCl (pH 8.0) in the bottom of each tube such that the pH was changed from the acid pH of glycine to the higher pH to stabilize the antibody. Peak protein fractions from the column were pooled, dialyzed against distilled $H_2O$ and used as whole antibody or were treated further to obtain Fab fragments.

To obtain Fab fragments of the individual monoclonal antibodies, 10 to 15 mg of the antibody purified using protein A columns, as described above, were lyophilized and then dissolved in 100 mM sodium phosphate, 10 mM cysteine, pH 7.2 (2 ml/20 mg antibody). Papain, 10-15 units per mg, was added to a ratio of 1:100, enzyme to antibody by weight. This reaction was incubated overnight for 16 hours at 37°C and it was terminated with the addition of iodoacetamide to a final concentration of 30 mM. The digested antibody was then chromatographed over a cross-linked dextran (Sephadex G-75) column (2.5 X 20 cm, width x height) at 4°C and the fractions were monitored for absorbance at 280 nm as a measure of the protein present.  The second peak off the column, which contained the majority of the Fab fragments, was pooled and then passed through a protein A-sepharose column to eliminate any contaminating Fc fragments or undigested antibody which bind to the protein A column.  The peak fractions of Fab fragments from the protein A column were pooled, dialyzed against distilled $H_2O$, and then lyophilized in 200 μg aliquots and stored at -80°C.

## EXAMPLE 17

### Crosslinking Monoclonal Antibodies to HRV

Neutralizing monoclonal antibodies directed towards HRV14 were cross-linked to the virus by the use of the heterobifunctional crosslinker toluene-2,4-diisocyanate (TDI), with the following minor adaptations of the procedure described in Emini et al. (J. Virol. 43:997-1005, 1982).  Sodium phosphate buffer (10 mM, pH 7.2) was added to dried Fab fragments of a monoclonal antibody, usually to a final concentration of 0.5 μg/μl.  TDI was then added, 1.1 μl/200 μl of antibody.  The antibody-Fab fragment was then incubated with the TDI at either 4°C or at room temperature.  The reaction was terminated by taking aliquots at various times, usually less than 10 minutes, and incubating these aliquots at 0°C for 10 minutes to solidify the TDI.  The TDI was removed by pelleting the solidified TDI at 2,000 x g at 0°C and then repeating the 0°C incubation and pelleting steps.  The final supernatant liquid then was taken and HRV14 was added to the antibody.  HRV was added either as $S^{35}$ labelled HRV, with 3000 to 7000 cpm.

The virus was incubated with the antibody for 30 minutes at room temperature.

Saturated tribasic phosphate buffer was added to raise the pH to 9.6 for 10 minutes at room temperature. Saturated monobasic phosphate was then added to lower the pH to 7.0. The samples then were dialyzed against $dH_2O$ overnight with 3 changes of the water. Each sample was lyophilized to dryness and run on a polyacrylamide gel (Laemmli, Nature 227: 680-685, 1970). Following the electrophoresis, the gel was dried and then exposed to X-ray film for varying lengths of time. To determine the degree of crosslinking to individual proteins, a densitometer tracing of the X-ray film was performed which also gave a quantitative measure of the various viral peaks. The neutralizing monoclonal antibodies were found to crosslink to VP1, VP2 or VP3.

0169146

## WHAT IS CLAIMED IS:

1.  DNA representative of genomic RNA of HRV Type 14 having the nucleotide and corresponding amino acid sequence:

```
TTAAAACAGCG GATGGGTATC CCACCATTCG ACCCATTGGG TGTAGTACTC
TGGTACTATG TACCTTTGTA CGCCTGTTTC TCCCCAACCA CCCTTCCTTA
AAATTCCCAC CCATGAAACG TTAGAAGCTT GACATTAAAG TACAATAGGT
GGCGCCATAT CCAATGGTGT CTATGTACAA GCACTTCTGT TTCCCAGGAG
CGAGGTAAAG GCTGTACCCA CTGCCAAAAG CCTTTAACCG TATCCGCCAA
CCAACTACGT AACAGTTAGT ACCATCTTGT TCTTGACTGG ACGTTCGATC
AGGTGGATTT TCCCTCCACT AGTTTGGTCG ATGAGGCTAG GAATTCCCCA
CGGGTGACCG TGTCCTAGCC TGCGTGGCGG CCAGACCCAG CTTATGCTGG
GACGCCCTTT TAAGGACATG GTGTGAAGAC TCGCATGTGC TTGGTTGTGA
GTCCTCCGGC CCCTGAATGC GGCTAACCTT AACCCTAGAG CCTTATGCCA
CGATCCAGTG GTTGTAAGGT CGTAATGAGC AATTCCGGGA CGGGACCGAC
TACTTTGGGT GTCCGTGTTT CTCATTTTTC TTCATATTGT CTTATGGTCA
CAGCATATAT ATACATATAC TGTGATC
```

VP4

```
ATG GGC GCT CAG GTT TCT ACA CAG AAA AGT GGA TCT CAC
MET Gly Ala Gln Val Ser Thr Gln Lys Ser Gly Ser His

GAA AAT CAA AAC ATT TTG ACC AAT GGA TCA AAT CAG ACT
Glu Asn Gln Asn Ile Leu Thr Asn Gly Ser Asn Gln Thr

TTC ACA GTT ATA AAT TAC TAT AAG GAT GCA GCA AGT ACA
Phe Thr Val Ile Asn Tyr Tyr Lys Asp Ala Ala Ser Thr

TCA TCA GCT GGT CAA TCA CTG TCA ATG GAC CCA TCT AAG
Ser Ser Ala Gly Gln Ser Leu Ser MET Asp Pro Ser Lys
```

```
        TTT ACA GAA CCA GTT AAA GAT CTC ATG CTT AAG GGT GCA
        Phe Thr Glu Pro Val Lys Asp Leu MET Leu Lys Gly Ala


            VP4 VP2
        CCA GCA TTG ATT TCA CCC AAT GTT GAG GCC TGT GGT TAT
        Pro Ala Leu Asn Ser Pro Asn Val Glu Ala Cys Gly Tyr


        AGT GAT AGA GTA CAA CAA ATC ACA CTC GGG AAT TCA ACA
        Ser Asp Arg Val Gln Gln Ile Thr Leu Gly Asn Ser Thr


        ATA ACA ACA CAA GAA GCA GCC AAC GCT GTT GTG TGT TAT
        Ile Thr Thr Gln Glu Ala Ala Asn Ala Val Val Cys Tyr


        GCT GAA TGG CCA GAG TAC CTT CCA GAT GTG GAC GCT AGT
        Ala Glu Trp Pro Glu Tyr Leu Pro Asp Val Asp Ala Ser


        GAT GTC AAT AAA ACT TCA AAA CCA GAC ACT TCT GTC TGT
        Asp Val Asn Lys Thr Ser Lys Pro Asp Thr Ser Val Cys


        AGG TTT TAC ACA TTG GAT AGT AAG ACA TGG ACA ACA GGT
        Arg Phe Tyr Thr Leu Asp Ser Lys Thr Trp Thr Thr Gly


        TCT AAA GGC TGG TGC TGG AAA TTA CCA GAT GCA CTC AAG
        Ser Lys Gly Trp Cys Trp Lys Leu Pro Asp Ala Leu Lys


        GAT ATG GGT GTG TTC GGG CAA AAC ATG TTT TTC TAC TCA
        Asp MET Gly Val Phe Gly Gln Asn MET Phe Phe Tyr Ser


        CTA GGA AGA TCA GGT TAC ACA GTA CAC GTT CAG TGC AAT
        Leu Gly Arg Ser Gly Tyr Thr Val His Val Gln Cys Asn
```

0169146

```
GCC ACA AAA TTC CAT AGC GGT TGT CTA CTT GTA GTT GTA
Ala Thr Lys Phe His Ser Gly Cys Leu Leu Val Val Val

ATA CCA GAA CAC CAA CTG GCT TCA CAT GAG GGT GGC AAT
Ile Pro Glu His Gln Leu Ala Ser His Glu Gly Gly Asn

GTT TCA GTT AAA TAC ACA TTC ACG CAT CCA GGT GAA CGT
Val Ser Val Lys Tyr Thr Phe Thr His Pro Gly Glu Arg

GGT ATA GAT TTA TCA TCT GCA AAT GAA GTG GGA GGG CCT
Gly Ile Asp Leu Ser Ser Ala Asn Glu Val Gly Gly Pro

GTC AAG GAT GTC ATA TAC AAT ATG AAT GGT ACT TTA TTA
Val Lys Asp Val Ile Tyr Asn MET Asn Gly Thr Leu Leu

GGA AAT CTG CTC ATT TTC CCT CAC CAG TTC ATT AAT CTA
Gly Asn Leu Leu Ile Phe Pro His Gln Phe Ile Asn Leu

AGA ACC AAT AAT ACA GCC ACA ATA GTG ATA CCA TAC ATA
Arg Thr Asn Asn Thr Ala Thr Ile Val Ile Pro Tyr Ile

AAC TCA GTA CCC ATT GAT TCA ATG ACA CGT CAC AAC AAT
Asn Ser Val Pro Ile Asp Ser MET Thr Arg His Asn Asn

GTC TCA CTG ATG GTC ATC CCT ATT GCC CCT CTT ACA GTA
Val Ser Leu MET Val Ile Pro Ile Ala Pro Leu Thr Val

CCA ACT GGA GCA ACT CCC TCA CTC CCT ATA ACA GTC ACA
Pro Thr Gly Ala Thr Pro Ser Leu Pro Ile Thr Val Thr
```

```
    ATA GCA CCT ATG TGC ACT GAG TTC TCT GGG ATA AGG TCC
    Ile Ala Pro MET Cys Thr Glu Phe Ser Gly Ile Arg Ser


                VP2 VP3
    AAG TCA ATT GTG CCA CAA GGT TTG CCA ACT ACA ACT TTG
    Lys Ser Ile Val Pro Gln Gly Leu Pro Thr Thr Thr Leu


    CCG GGG TCA GGA CAA TTC TTG ACC ACA GAT GAC AGG CAA
    Pro Gly Ser Gly Gln Phe Leu Thr Thr Asp Asp Arg Gln


    TCC CCC AGT GCA CTG CCA AAT TAT GAG CCA ACT CCA AGA
    Ser Pro Ser Ala Leu Pro Asn Tyr Glu Pro Thr Pro Arg


    ATA CAC ATA CTA GGG AAA GTT CAT AAC TTG CTA GAA ATT
    Ile His Ile Leu Gly Lys Val His Asn Leu Leu Glu Ile


    ATA CAG GTA GAT ACA CTC ATT CCT ATG AAC AAC ACG CAT
    Ile Gln Val Asp Thr Leu Ile Pro MET Asn Asn Thr His


    ACA AAA GAT GAG GTT AAC AGT TAC CTC ATA CCA CTA AAT
    Thr Lys Asp Glu Val Asn Ser Tyr Leu Ile Pro Leu Asn


    GCA AAC AGG CAA AAT GAG CAG GTT TTT GGG ACA AAC CTG
    Ala Asn Arg Gln Asn Glu Gln Val Phe Gly Thr Asn Leu


    TTT ATT GGT GAT GGG GTC TTC AAA ACT ACT CTT CTG GGT
    Phe Ile Gly Asp Gly Val Phe Lys Thr Thr Leu Leu Gly


    GAA ATT GTT CAG TAC TAT ACA CAT TGG TCT GGA TCA CTT
    Glu Ile Val Gln Tyr Tyr Thr His Trp Ser Gly Ser Leu
```

```
    AGA TTC TCT TCG ATG TAT ACT GGT CCT GCC TTG TCC AGT
    Arg Phe Ser Ser MET Tyr Thr Gly Pro Ala Leu Ser Ser


    GCT AAA CTC ACT CTA GCA TAC ACC CCG CCT GGT GCT CGT
    Ala Lys Leu Thr Leu Ala Tyr Thr Pro Pro Gly Ala Arg


    GGT CCA CAG GAC AGG AGA GAA GCA ATG CTA GGT ACT CAT
    Gly Pro Gln Asp Arg Arg Glu Ala MET Leu Gly Thr His


    GTT GTC TGG GAT ATT GGT CTG CAA TCC ACC ATA GTA ATG
    Val Val Trp Asp Ile Gly Leu Gln Ser Thr Ile Val MET


    ACA ATA CCA TGG ACA TCA GGG GTG CAG TTT AGA TAT ACT
    Thr Ile Pro Trp Thr Ser Gly Val Gln Phe Arg Tyr Thr


    GAT CCA GAT ACA TAC ACC AGT GCT GGC TTT CTA TCA TGT
    Asp Pro Asp Thr Tyr Thr Ser Ala Gly Phe Leu Ser Cys


    TGG TAT CAA ACT TCT CTT ATA CTT CCC CCA GAA ACG ACC
    Trp Tyr Gln Thr Ser Leu Ile Leu Pro Pro Glu Thr Thr


    GGC CAG GTC TAC TTA TTA TCA TTC ATA AGT GCA TGT CCA
    Gly Gln Val Tyr Leu Leu Ser Phe Ile Ser Ala Cys Pro


    GAT TTT AAG CTT AGG CTG ATG AAA GAT ACT CAA ACT ATC
    Asp Phe Lys Leu Arg Leu MET Lys Asp Thr Gln Thr Ile


                            VP3 VP1
    TCA CAG ACT GTT GCA CTC ACT GAA GGC TTA GGT GAT GAA
    Ser Gln Thr Val Ala Leu Thr Glu Gly Leu Gly Asp Glu
```

TTA GAA GAA GTC ATC GTT GAG AAA ACG AAA CAG ACG GTG
Leu Glu Glu Val Ile Val Glu Lys Thr Lys Gln Thr Val


GCC TCA ATC TCA TCT GGT CCA AAA CAC ACA CAA AAA GTC
Ala Ser Ile Ser Ser Gly Pro Lys His Thr Gln Lys Val


CCC ATA CTA ACT GCA AAC GAA ACA GGG GCC ACA ATG CCT
Pro Ile Leu Thr Ala Asn Glu Thr Gly Ala Thr MET Pro


GTT CTT CCA TCA GAC AGC ATA GAA ACC AGA ACT ACC TAC
Val Leu Pro Ser Asp Ser Ile Glu Thr Arg Thr Thr Tyr


ATG CAC TTT AAT GGT TCA GAA ACT GAT GTA GAA TGC TTT
MET His Phe Asn Gly Ser Glu Thr Asp Val Glu Cys Phe


TTG GGT GGT GCG GCT TGT GTG CAT GTA ACT GAA ATA CAA
Leu Gly Gly Ala Ala Cys Val His Val Thr Glu Ile Gln


AAC AAA GAT GCT ACT GGA ATA GAT AAT CAC AGA GAA GCA
Asn Lys Asp Ala Thr Gly Ile Asp Asn His Arg Glu Ala


AGA TTG TTC AAT GAT TGG AAA ATC AAC CTG TCC AGC CTT
Arg Leu Phe Asn Asp Trp Lys Ile Asn Leu Ser Ser Leu


GTC CAA CTT AGA AAG AAA CTG GAA CTC TTC ACT TAT GTT
Val Gln Leu Arg Lys Lys Leu Glu Leu Phe Thr Tyr Val


AGG TTT GAT TCT GAG TAT ACC ATA CTG GCC ACT GCA TCT
Arg Phe Asp Ser Glu Tyr Thr Ile Leu Ala Thr Ala Ser


CAA CCT GAT TCA GCA AAC TAT TCA AGC AAT TTG GTG GTC
Gln Pro Asp Ser Ala Asn Tyr Ser Ser Asn Leu Val Val

```
CAA GCC ATG TAT GTT CCA CAT GGT GCC CCG AAA TCC AAA
Gln Ala MET Tyr Val Pro His Gly Ala Pro Lys Ser Lys

AGA GTG GAC GAT TAC ACA TGG CAA AGT GCT TCA AAC CCC
Arg Val Asp Asp Tyr Thr Trp Gln Ser Ala Ser Asn Pro

AGT GTA TTC TTC AAG GTG GGG GAT ACA TCA AGG TTT AGT
Ser Val Phe Phe Lys Val Gly Asp Thr Ser Arg Phe Ser

GTG CCT TAT GTA GGA TTG GCA TCA GCA TAT AAT TGT TTT
Val Pro Tyr Val Gly Leu Ala Ser Ala Tyr Asn Cys Phe

TAT GAT GGT TAC TCA CAT GAT GAT GCA GAA ACT CAG TAT
Tyr Asp Gly Tyr Ser His Asp Asp Ala Glu Thr Gln Tyr

GGC ATA ACT GTT CTA AAC CAT ATG GGT AGT ATG GCA TTC
Gly Ile Thr Val Leu Asn His MET Gly Ser MET Ala Phe

AGA ATA GTA AAT GAA CAT GAT GAA CAC TTA ACT CTT GTC
Arg Ile Val Asn Glu His Asp Glu His Leu Thr Leu Val

AAG ATC AGA GTT TAT CAC AGG GCA AAG CTC GTT GAA GCA
Lys Ile Arg Val Tyr His Arg Ala Lys Leu Val Glu Ala

TGG ACT CCC AGA GCA CCC AGA GCA CTA CCC TAC ACA TCA
Trp Thr Pro Arg Ala Pro Arg Ala Leu Pro Tyr Thr Ser

ATA GGG CGC ACA AAT TAT CCT AAG AAT ACA GAA CCA GTA
Ile Gly Arg Thr Asn Tyr Pro Lys Asn Thr Glu Pro Val
```

VP1 3B

```
ATT AAG AAG AGG AAA GGT GAC ATT AAA TCC TAT GGT TTA
Ile Lys Lys Arg Lys Gly Asp Ile Lys Ser Tyr Gly Leu

GGA CCT AGG TAC GGT GGG ATT TAT ACA TCA AAT GTT AAA
Gly Pro Arg Tyr Gly Gly Ile Tyr Thr Ser Asn Val Lys

ATA ATG AAT TAC CAC TTG ATG ACA CCA GAA GAC CAC CAT
Ile MET Asn Tyr His Leu MET Thr Pro Glu Asp His His

AAT CTG ATA GCA CCC TAT CCA AAT AGA GAT TTA GCA ATA
Asn Leu Ile Ala Pro Tyr Pro Asn Arg Asp Leu Ala Ile

GTC TCA ACA GGA GGA CAT GGT GCA GAA ACA ATA CCA CAC
Val Ser Thr Gly Gly His Gly Ala Glu Thr Ile Pro His

TGT AAC CGT ACA TCA GGT GTT TAC TAT TCC ACA TAT TAC
Cys Asn Arg Thr Ser Gly Val Tyr Tyr Ser Thr Tyr Tyr

AGA AAG TAT TAC CCC ATA ATT TGC GAA AGC CCG CCA ACA
Arg Lys Tyr Tyr Pro Ile Ile Cys Glu Ser Pro Pro Thr

TCT GAA TTG GGG AAG CCC TTA TTA CCC AAG CAG ATT CAA
Ser Glu Leu Gly Lys Pro Leu Leu Pro Lys Gln Ile Gln

GCA GGA GTG ATG AAA GGG GTT GGG CCG GCA GAG CTA GGA
Ala Gly Val MET Lys Gly Val Gly Pro Ala Glu Leu Gly

GAC TGC GGT GGG ATT TTG AGA TGC ATA CAT GGT CCC ATT
Asp Cys Gly Gly Ile Leu Arg Cys Ile His Gly Pro Ile

GGA TTG TTA ACA GCT GAA GGT AGT GGA TAT GTT TGT TTT
Gly Leu Leu Thr Ala Glu Gly Ser Gly Tyr Val Cys Phe
```

```
                                                    3B
        GCT GAC ATA CGA CAG TTG GAG TGT ATC GCA GAG GAA CAG
        Ala Asp Ile Arg Gln Leu Glu Cys Ile Ala Glu Glu Gln

        5B
        GGG CTG AGT GAT TAC ATC ACA GGT TTG GGT AGA GCT TTT
        Gly Leu Ser Asp Tyr Ile Thr Gly Leu Gly Arg Ala Phe

        GGT GTC GGG TTC ACT GAC CAA ATC TCA ACA AAA GTC ACA
        Gly Val Gly Phe Thr Asp Gln Ile Ser Thr Lys Val Thr

        GAA CTA CAA GAA GTG GCG AAA GAT TTC CTC ACC ACA AAA
        Glu Leu Gln Glu Val Ala Lys Asp Phe Leu Thr Thr Lys

        GTT TTG TCC AAA GTG GTC AAA ATG GTT TCA GCT TTA GTG
        Val Leu Ser Lys Val Val Lys MET Val Ser Ala Leu Val

        ATC ATT TGC AGA AAT CAT GAT GAC TTG GTC ACT GTT ACG
        Ile Ile Cys Arg Asn His Asp Asp Leu Val Thr Val Thr

        GCC ACT CTA GCA CTA CTT GGA TGT GAT GGA TCT CCT TGG
        Ala Thr Leu Ala Leu Leu Gly Cys Asp Gly Ser Pro Trp

        AGA TTT CTG AAG ATG TAC ATT TCC AAA CAC TTT CAG GTG
        Arg Phe Leu Lys MET Tyr Ile Ser Lys His Phe Gln Val

                            5B X
        CCT TAC ATT GAA AGA CAA GCA AAT GAT GGA TGG TTC AGA
        Pro Tyr Ile Glu Arg Gln Ala Asn Asp Gly Trp Phe Arg
```

```
AAG TTT AAT GAT GCA TGT AAT GCT GCA AAG GGA TTG GAA
Lys Phe Asn Asp Ala Cys Asn Ala Ala Lys Gly Leu Glu


TGG ATT GCT AAT AAG ATT TCC AAA CTG ATT GAA TGG ATA
Trp Ile Ala Asn Lys Ile Ser Lys Leu Ile Glu Trp Ile


AAA AAC AAA GTA CTT CCC CAA GCC AAA GAA AAA CTA GAA
Lys Asn Lys Val Leu Pro Gln Ala Lys Glu Lys Leu Glu


TTT TGT AGT AAA CTC AAA CAA CTT GAT ATA CTA GAG AGA
Phe Cys Ser Lys Leu Lys Gln Leu Asp Ile Leu Glu Arg


CAA ATA ACC ACC ATG CAT ATC TCG AAT CCA ACA CAG GAA
Gln Ile Thr Thr MET His Ile Ser Asn Pro Thr Gln Glu


AAA CGA GAG CAG TTG TTC AAT AAC GTA TTG TGG TTG GAA
Lys Arg Glu Gln Leu Phe Asn Asn Val Leu Trp Leu Glu


CAA ATG TCG CAA AAG TTT GCC CCA TTT TAT GCC GTT GAA
Gln MET Ser Gln Lys Phe Ala Pro Phe Tyr Ala Val Glu


TCA AAA AGA ATC AGG GAA CTC AAG AAC AAA ATG GTA AAT
Ser Lys Arg Ile Arg Glu Leu Lys Asn Lys MET Val Asn


TAT ATG CAA TTT AAA AGT AAA CAA AGA ACT GAA CCA GTG
Tyr MET Gln Phe Lys Ser Lys Gln Arg Thr Glu Pro Val


TGT GTA TTA ATC CAT GGT ACA CCC GGT TCT GGT AAA TCA
Cys Val Leu Ile His Gly Thr Pro Gly Ser Gly Lys Ser


TTA ACA ACA TCC ATT GTG GGA CGT GCA ATT GCA GAA CAC
Leu Thr Thr Ser Ile Val Gly Arg Ala Ile Ala Glu His
```

```
TTC AAT TCA GCA GTA TAT TCA CTT CCA CCA GAT CCC AAG
Phe Asn Ser Ala Val Tyr Ser Leu Pro Pro Asp Pro Lys

CAC TTT GAT GGT TAT CAG CAA CAG GAA GTT GTG ATT ATG
His Phe Asp Gly Tyr Gln Gln Gln Glu Val Val Ile MET

GAT GAT CTG AAC CAA AAT CCA GAT GGA CAG GAT ATA AGC
Asp Asp Leu Asn Gln Asn Pro Asp Gly Gln Asp Ile Ser

ATG TTT TGT CAA ATG GTT TCT TCA GTG GAT TTC TTG CCT
MET Phe Cys Gln MET Val Ser Ser Val Asp Phe Leu Pro

CCA ATG GCT AGT TTA GAT AAC AAG GGC ATG TTA TTC ACC
Pro MET Ala Ser Leu Asp Asn Lys Gly MET Leu Phe Thr

AGT AAT TTT GTT CTA GCC TCC ACA AAT TCT AAC ACA CTA
Ser Asn Phe Val Leu Ala Ser Thr Asn Ser Asn Thr Leu

AGC CCC CCA ACA ATC TTG AAT CCT GAA GCT TTA GTC AGG
Ser Pro Pro Thr Ile Leu Asn Pro Glu Ala Leu Val Arg

AGA TTT GGT TTT GAC CTA GAT ATA TGT TTG CAT ACT ACC
Arg Phe Gly Phe Asp Leu Asp Ile Cys Leu His Thr Thr

TAC ACA AAG AAT GGA AAA CTC AAT GCA GGC ATG TCA ACC
Tyr Thr Lys Asn Gly Lys Leu Asn Ala Gly MET Ser Thr

AAG ACA TGC AAA GAT TGC CAT CAA CCA TCT AAT TTC AAG
Lys Thr Cys Lys Asp Cys His Gln Pro Ser Asn Phe Lys
```

AAA TGT TGC CCC CTA GTC TGT GGA AAA GCT ATT AGC TTG
Lys Cys Cys Pro Leu Val Cys Gly Lys Ala Ile Ser Leu

GTA GAC AGA ACT ACC AAC GTT AGG TAT AGT GTG GAT CAA
Val Asp Arg Thr Thr Asn Val Arg Tyr Ser Val Asp Gln

CTG GTC ACG GCT ATT ATA AGT GAT TTC AAG AGC AAA ATG
Leu Val Thr Ala Ile Ile Ser Asp Phe Lys Ser Lys MET

X 1B
CAA ATT ACA GAT TCC CTA GAA ACA CTG TTT CAA GGA CCA
Gln Ile Thr Asp Ser Leu Glu Thr Leu Phe Gln Gly Pro

GTG TAT AAA GAT TTA GAG ATT GAT GTT TGC AAC ACA CCA
Val Tyr Lys Asp Leu Glu Ile Asp Val Cys Asn Thr Pro

CCT TCA GAA TGT ATC AAC GAT TTA CTG AAA TCT GTA GAT
Pro Ser Glu Cys Ile Asn Asp Leu Leu Lys Ser Val Asp

TCA GAA GAG ATT AGG GAA TAT TGT AAG AAG AAG AAA TGG
Ser Glu Glu Ile Arg Glu Tyr Cys Lys Lys Lys Lys Trp

ATT ATA CCT GAA ATT CCT ACC AAC ATA GAA AGG GCT ATG
Ile Ile Pro Glu Ile Pro Thr Asn Ile Glu Arg Ala MET

AAT CAA GCC AGC ATT ATT ATT AAT ACT ATT CTG ATG TTT
Asn Gln Ala Ser Ile Ile Ile Asn Thr Ile Leu MET Phe

GTC AGT ACA TTA GGT ATT GTT TAT GTC ATT TAT AAA TTG
Val Ser Thr Leu Gly Ile Val Tyr Val Ile Tyr Lys Leu

1B VPg
TTT GCT CAA ACT CAA GGA CCA TAT TCT GGT AAC CCG CCT
Phe Ala Gln Thr Gln Gly Pro Tyr Ser Gly Asn Pro Pro

CAC AAT AAA CTA AAA GCC CCA ACT TTA CGC CCA GTT GTT
His Asn Lys Leu Lys Ala Pro Thr Leu Arg Pro Val Val


VPg Protease
GTG CAA GGA CCA AAC ACA GAA TTT GCA CTA TCC CTG TTA
Val Gln Gly Pro Asn Thr Glu Phe Ala Leu Ser Leu Leu


AGG AAA AAC ATA ATG ACT ATA ACA ACC TCA AAG GGA GAG
Arg Lys Asn Ile MET Thr Ile Thr Thr Ser Lys Gly Glu


TTC ACA GGG TTA GGC ATA CAT GAT CGT GTC TGT GTG ATA
Phe Thr Gly Leu Gly Ile His Asp Arg Val Cys Val Ile


CCC ACA CAC GCA CAG CCT GGT GAT GAT GTA CTA GTG AAT
Pro Thr His Ala Gln Pro Gly Asp Asp Val Leu Val Asn


GGT CAG AAA ATT AGA GTT AAG GAT AAG TAC AAA TTA GTA
Gly Gln Lys Ile Arg Val Lys Asp Lys Tyr Lys Leu Val


GAT CCA GAG AAC ATT AAT CTA GAG CTT ACA GTG TTG ACT
Asp Pro Glu Asn Ile Asn Leu Glu Leu Thr Val Leu Thr


TTA GAT AGA AAT GAA AAA TTC AGA GAT ATC AGG GGA TTT
Leu Asp Arg Asn Glu Lys Phe Arg Asp Ile Arg Gly Phe


ATA TCA GAA GAT CTA GAA GGT GTG GAT GCC ACT TTG GTA
Ile Ser Glu Asp Leu Glu Gly Val Asp Ala Thr Leu Val

GTA CAT TCA AAT AAC TTT ACC AAC ACT ATC TTA GAA GTT
Val His Ser Asn Asn Phe Thr Asn Thr Ile Leu Glu Val

GGC CCT GTA ACA ATG GCA GGA CTT ATT AAT TTG AGT AGC
Gly Pro Val Thr MET Ala Gly Leu Ile Asn Leu Ser Ser

ACC CCC ACT AAC AGA ATG ATT CGT TAT GAT TAT GCA ACA
Thr Pro Thr Asn Arg MET Ile Arg Tyr Asp Tyr Ala Thr

AAA ACT GGG CAG TGT GGA GGT GTG CTG TGT GCT ACT GGT
Lys Thr Gly Gln Cys Gly Gly Val Leu Cys Ala Thr Gly

AAG ATC TTT GGT ATT CAT GTT GGC GGT AAT GGA AGA CAA
Lys Ile Phe Gly Ile His Val Gly Gly Asn Gly Arg Gln

GGA TTT TCA GCT CAA CTT AAA AAA CAA TAT TTT GTA GAG
Gly Phe Ser Ala Gln Leu Lys Lys Gln Tyr Phe Val Glu

Protease Replicase
AAA CAA  GGC CAA GTA ATA GCT AGA CAT AAG GTT AGG GAG
Lys Gln  Gly Gln Val Ile Ala Arg His Lys Val Arg Glu

TTT AAC ATA AAT TCA GTC AAC ACG GCA ACT AAG TCA AAA
Phe Asn Ile Asn Ser Val Asn Thr Ala Thr Lys Ser Lys

TTA CAT CCC AGT GTA TTT TAT GAT GTT TTT CCA GGT GAC
Leu His Pro Ser Val Phe Tyr Asp Val Phe Pro Gly Asp

AAG GAA CCT GCT GTA TTG AGT GAC AAT GAT CCC AGA CTG
Lys Glu Pro Ala Val Leu Ser Asp Asn Asp Pro Arg Leu

```
GAA GTT AAA TTG ACT GAA TCA TTA TTC TCT AAG TAC AAG
Glu Val Lys Leu Thr Glu Ser Leu Phe Ser Lys Tyr Lys


GGG AAT GTA AAT ACG GAA CCC ACT GAA AAT ATG CTT GTG
Gly Asn Val Asn Thr Glu Pro Thr Glu Asn MET Leu Val


GCT GTA GAC CAT TAT GCA GGG CAA CTA TTA TCA CTA GAT
Ala Val Asp His Tyr Ala Gly Gln Leu Leu Ser Leu Asp


ATC CCC ACT TCT GAA CTT ACA CTA AAA GAA·GCA TTA TAT
Ile Pro Thr Ser Glu Leu Thr Leu Lys Glu Ala Leu Tyr


GGA GTA GAT GGA CTA GAA CCT ATA GAT ATT ACA ACC AGT
Gly Val Asp Gly Leu Glu Pro Ile Asp Ile Thr Thr Ser


GCA GGA TTT CCC TAT GTG AGT CTT GGG ATC AAA AAG AGA
Ala Gly Phe Pro Tyr Val Ser Leu Gly Ile Lys Lys Arg


GAC ATT CTG AAT AAA GAG ACC CAG GAC ACA GAA AAG ATG
Asp Ile Leu Asn Lys Glu Thr Gln Asp Thr Glu Lys MET


AAG TTT TAT CTA GAC AAG TAT GGC ATT GAC TTG CCT CTA
Lys Phe Tyr Leu Asp Lys Tyr Gly Ile Asp Leu Pro Leu


GTT ACA TAT ATT AAG GAT GAA TTA AGA AGT GTT GAC AAA
Val Thr Tyr Ile Lys Asp Glu Leu Arg Ser Val Asp Lys


GTC CGA TTA GGG AAA AGT AGA TTA ATT GAA GCC TCC AGT
Val Arg Leu Gly Lys Ser Arg Leu Ile Glu Ala Ser Ser


TTG AAT GAT TCT GTT AAC ATG AGA ATG AAA CTA GGC AAC
Leu Asn Asp Ser Val Asn MET Arg MET Lys Leu Gly Asn
```

0169146

```
CTT TAC AAA GCA TTC CAT CAA AAT CCC GGT GTT CTG ACT
Leu Tyr Lys Ala Phe His Gln Asn Pro Gly Val Leu Thr


GGA TCA GCA GTG GGT TGT GAT CCT GAT GTG TTT TGG TCT
Gly Ser Ala Val Gly Cys Asp Pro Asp Val Phe Trp Ser


GTC ATC CCT TGC TTA ATG GAT GGG CAC CTG ATG GCA TTT
Val Ile Pro Cys Leu MET Asp Gly His Leu MET Ala Phe


GAT TAC TCT AAT TTT GAT GCC TCT TTG TCA CCA GTT TGG
Asp Tyr Ser Asn Phe Asp Ala Ser Leu Ser Pro Val Trp


TTT GTC TGT CTA GAG AAG GTT TTG ACC AAG TTA GGC TTT
Phe Val Cys Leu Glu Lys Val Leu Thr Lys Leu Gly Phe


GCA GGC TCT TCA TTA ATT CAA TCA ATT TGT AAT ACC CAT
Ala Gly Ser Ser Leu Ile Gln Ser Ile Cys Asn Thr His


CAT ATC TTT AGG GAT GAA ATA TAT GTG GTT GAA GGT GGC
His Ile Phe Arg Asp Glu Ile Tyr Val Val Glu Gly Gly


ATG CCC TCA GGG TGT TCA GGA ACC AGC ATA TTC AAT TCC
MET Pro Ser Gly Cys Ser Gly Thr Ser Ile Phe Asn Ser


ATG ATC AAC AAC ATA ATC ATT AGG ACT TTG ATA TTA GAT
MET Ile Asn Asn Ile Ile Ile Arg Thr Leu Ile Leu Asp


GCA TAT AAA GGA ATA GAT TTA GAC AAA CTT AAA ATC TTA
Ala Tyr Lys Gly Ile Asp Leu Asp Lys Leu Lys Ile Leu
```

GCT TAC GGT GAT GAT TTG ATT GTT TCT TAT CCT TAT GAA
Ala Tyr Gly Asp Asp Leu Ile Val Ser Tyr Pro Tyr Glu


CTG GAT CCA CAA GTG TTG GCA ACT CTT GGT AAA AAT TAT
Leu Asp Pro Gln Val Leu Ala Thr Leu Gly Lys Asn Tyr


GGA CTA ACC ATC ACA CCC CCA GAC AAA TCT GAA ACT TTT
Gly Leu Thr Ile Thr Pro Pro Asp Lys Ser Glu Thr Phe


ACA AAA ATG ACA TGG GAA AAC TTG ACA TTT TTA AAG AGA
Thr Lys MET Thr Trp Glu Asn Leu Thr Phe Leu Lys Arg


TAC TTC AAG CCT GAT CAA CAA TTT CCC TTT TTG GTT CAC
Tyr Phe Lys Pro Asp Gln Gln Phe Pro Phe Leu Val His


CCA GTT ATG CCC ATG AAA GAT ATA CAT GAG TCA ATC AGA
Pro Val MET Pro MET Lys Asp Ile His Glu Ser Ile Arg


TGG ACA AAG GAT CCT AAA AAC ACA CAG GAT CAC GTC CGA
Trp Thr Lys Asp Pro Lys Asn Thr Gln Asp His Val Arg


TCA TTA TGC ATG TTA GCA TGG CAC TCA GGA GAA AAA GAG
Ser Leu Cys MET Leu Ala Trp His Ser Gly Glu Lys Glu


TAC AAT GAA TTC ATT CAG AAG ATC AGA ACT ACT GAC ATT
Tyr Asn Glu Phe Ile Gln Lys Ile Arg Thr Thr Asp Ile


GGA AAA TGT CTA ATT CTC CCA GAA TAC AGC GTA CTT AGG
Gly Lys Cys Leu Ile Leu Pro Glu Tyr Ser Val Leu Arg

Replicase

AGG CGC TGG TTG GAC CTC TTT
Arg Arg Trp Leu Asp Leu Phe


TAGGTTAACAATATAGACACTTAATTTGAGTAGAAGTAGGAGT


TTATAAAAAA AAAAAAAAA


2.  DNA representative of genomic RNA of VP4
of HRV Type 14 having the nucleotide and
corresponding amino acid sequence:
VP4
ATG GGC GCT CAG GTT TCT ACA CAG AAA AGT GGA TCT CAC
MET Gly Ala Gln Val Ser Thr Gln Lys Ser Gly Ser His


GAA AAT CAA AAC ATT TTG ACC AAT GGA TCA AAT CAG ACT
Glu Asn Gln Asn Ile Leu Thr Asn Gly Ser Asn Gln Thr


TTC ACA GTT ATA AAT TAC TAT AAG GAT GCA GCA AGT ACA
Phe Thr Val Ile Asn Tyr Tyr Lys Asp Ala Ala Ser Thr


TCA TCA GCT GGT CAA TCA CTG TCA ATG GAC CCA TCT AAG
Ser Ser Ala Gly Gln Ser Leu Ser MET Asp Pro Ser Lys


TTT ACA GAA CCA GTT AAA GAT CTC ATG CTT AAG GGT GCA
Phe Thr Glu Pro Val Lys Asp Leu MET Leu Lys Gly Ala


           VP4
CCA GCA TTG ATT
Pro Ala Leu Asn

0169146

3. DNA representative of genomic RNA of VP2 of HRV Type 14 having the nucleotide and corresponding amino acid sequence:

VP2

```
TCA CCC AAT GTT GAG GCC TGT GGT TAT
Ser Pro Asn Val Glu Ala Cys Gly Tyr


AGT GAT AGA GTA CAA CAA ATC ACA CTC GGG AAT TCA ACA
Ser Asp Arg Val Gln Gln Ile Thr Leu Gly Asn Ser Thr


ATA ACA ACA CAA GAA GCA GCC AAC GCT GTT GTG TGT TAT
Ile Thr Thr Gln Glu Ala Ala Asn Ala Val Val Cys Tyr


GCT GAA TGG CCA GAG TAC CTT CCA GAT GTG GAC GCT AGT
Ala Glu Trp Pro Glu Tyr Leu Pro Asp Val Asp Ala Ser


GAT GTC AAT AAA ACT TCA AAA CCA GAC ACT TCT GTC TGT
Asp Val Asn Lys Thr Ser Lys Pro Asp Thr Ser Val Cys


AGG TTT TAC ACA TTG GAT AGT AAG ACA TGG ACA ACA GGT
Arg Phe Tyr Thr Leu Asp Ser Lys Thr Trp Thr Thr Gly


TCT AAA GGC TGG TGC TGG AAA TTA CCA GAT GCA CTC AAG
Ser Lys Gly Trp Cys Trp Lys Leu Pro Asp Ala Leu Lys


GAT ATG GGT GTG TTC GGG CAA AAC ATG TTT TTC TAC TCA
Asp MET Gly Val Phe Gly Gln Asn MET Phe Phe Tyr Ser


CTA GGA AGA TCA GGT TAC ACA GTA CAC GTT CAG TGC AAT
Leu Gly Arg Ser Gly Tyr Thr Val His Val Gln Cys Asn
```

GCC ACA AAA TTC CAT AGC GGT TGT CTA CTT GTA GTT GTA
Ala Thr Lys Phe His Ser Gly Cys Leu Leu Val Val Val

ATA CCA GAA CAC CAA CTG GCT TCA CAT GAG GGT GGC AAT
Ile Pro Glu His Gln Leu Ala Ser His Glu Gly Gly Asn

GTT TCA GTT AAA TAC ACA TTC ACG CAT CCA GGT GAA CGT
Val Ser Val Lys Tyr Thr Phe Thr His Pro Gly Glu Arg

GGT ATA GAT TTA TCA TCT GCA AAT GAA GTG GGA GGG CCT
Gly Ile Asp Leu Ser Ser Ala Asn Glu Val Gly Gly Pro

GTC AAG GAT GTC ATA TAC AAT ATG AAT GGT ACT TTA TTA
Val Lys Asp Val Ile Tyr Asn MET Asn Gly Thr Leu Leu

GGA AAT CTG CTC ATT TTC CCT CAC CAG TTC ATT AAT CTA
Gly Asn Leu Leu Ile Phe Pro His Gln Phe Ile Asn Leu

AGA ACC AAT AAT ACA GCC ACA ATA GTG ATA CCA TAC ATA
Arg Thr Asn Asn Thr Ala Thr Ile Val Ile Pro Tyr Ile

AAC TCA GTA CCC ATT GAT TCA ATG ACA CGT CAC AAC AAT
Asn Ser Val Pro Ile Asp Ser MET Thr Arg His Asn Asn

GTC TCA CTG ATG GTC ATC CCT ATT GCC CCT CTT ACA GTA
Val Ser Leu MET Val Ile Pro Ile Ala Pro Leu Thr Val

CCA ACT GGA GCA ACT CCC TCA CTC CCT ATA ACA GTC ACA
Pro Thr Gly Ala Thr Pro Ser Leu Pro Ile Thr Val Thr

ATA GCA CCT ATG TGC ACT GAG TTC TCT GGG ATA AGG TCC
Ile Ala Pro MET Cys Thr Glu Phe Ser Gly Ile Arg Ser

                              VP2
AAG TCA ATT GTG CCA CAA
Lys Ser Ile Val Pro Gln


          4.  DNA representative of genomic RNA of VP1
of HRV type 14 having the nucleotide and
corresponding amino acid sequence:
VP1
GGC TTA GGT GAT GAA
Gly Leu Gly Asp Glu


TTA GAA GAA GTC ATC GTT GAG AAA ACG AAA CAG ACG GTG
Leu Glu Glu Val Ile Val Glu Lys Thr Lys Gln Thr Val

GCC TCA ATC TCA TCT GGT CCA AAA CAC ACA CAA AAA GTC
Ala Ser Ile Ser Ser Gly Pro Lys His Thr Gln Lys Val


CCC ATA CTA ACT GCA AAC GAA ACA GGG GCC ACA ATG CCT
Pro Ile Leu Thr Ala Asn Glu Thr Gly Ala Thr MET Pro


GTT CTT CCA TCA GAC AGC ATA GAA ACC AGA ACT ACC TAC
Val Leu Pro Ser Asp Ser Ile Glu Thr Arg Thr Thr Tyr


ATG CAC TTT AAT GGT TCA GAA ACT GAT GTA GAA TGC TTT
MET His Phe Asn Gly Ser Glu Thr Asp Val Glu Cys Phe


TTG GGT GGT GCG GCT TGT GTG CAT GTA ACT GAA ATA CAA
Leu Gly Gly Ala Ala Cys Val His Val Thr Glu Ile Gln


AAC AAA GAT GCT ACT GGA ATA GAT AAT CAC AGA GAA GCA
Asn Lys Asp Ala Thr Gly Ile Asp Asn His Arg Glu Ala

```
AGA TTG TTC AAT GAT TGG AAA ATC AAC CTG TCC AGC CTT
Arg Leu Phe Asn Asp Trp Lys Ile Asn Leu Ser Ser Leu

GTC CAA CTT AGA AAG AAA CTG GAA CTC TTC ACT TAT GTT
Val Gln Leu Arg Lys Lys Leu Glu Leu Phe Thr Tyr Val

AGG TTT GAT TCT GAG TAT ACC ATA CTG GCC ACT GCA TCT
Arg Phe Asp Ser Glu Tyr Thr Ile Leu Ala Thr Ala Ser

CAA CCT GAT TCA GCA AAC TAT TCA AGC AAT TTG GTG GTC
Gln Pro Asp Ser Ala Asn Tyr Ser Ser Asn Leu Val Val

CAA GCC ATG TAT GTT CCA CAT GGT GCC CCG AAA TCC AAA
Gln Ala MET Tyr Val Pro His Gly Ala Pro Lys Ser Lys

AGA GTG GAC GAT TAC ACA TGG CAA AGT GCT TCA AAC CCC
Arg Val Asp Asp Tyr Thr Trp Gln Ser Ala Ser Asn Pro

AGT GTA TTC TTC AAG GTG GGG GAT ACA TCA AGG TTT AGT
Ser Val Phe Phe Lys Val Gly Asp Thr Ser Arg Phe Ser

GTG CCT TAT GTA GGA TTG GCA TCA GCA TAT AAT TGT TTT
Val Pro Tyr Val Gly Leu Ala Ser Ala Tyr Asn Cys Phe

TAT GAT GGT TAC TCA CAT GAT GAT GCA GAA ACT CAG TAT
Tyr Asp Gly Tyr Ser His Asp Asp Ala Glu Thr Gln Tyr

GGC ATA ACT GTT CTA AAC CAT ATG GGT AGT ATG GCA TTC
Gly Ile Thr Val Leu Asn His MET Gly Ser MET Ala Phe
```

AGA ATA GTA AAT GAA CAT GAT GAA CAC TTA ACT CTT GTC
Arg Ile Val Asn Glu His Asp Glu His Leu Thr Leu Val

AAG ATC AGA GTT TAT CAC AGG GCA AAG CTC GTT GAA GCA
Lys Ile Arg Val Tyr His Arg Ala Lys Leu Val Glu Ala

TGG ACT CCC AGA GCA CCC AGA GCA CTA CCC TAC ACA TCA
Trp Thr Pro Arg Ala Pro Arg Ala Leu Pro Tyr Thr Ser

ATA GGG CGC ACA AAT TAT CCT AAG AAT ACA ·GAA CCA GTA
Ile Gly Arg Thr Asn Tyr Pro Lys Asn Thr Glu Pro Val

                                              VP1
ATT AAG AAG AGG AAA GGT GAC ATT AAA TCC TAT
Ile Lys Lys Arg Lys Gly Asp Ile Lys Ser Tyr

    5.   DNA representative of genomic RNA of
protease of HRV type 14 having the nucleotide and
corresponding amino acid sequence:
Protease
GGA CCA AAC ACA GAA TTT GCA CTA TCC CTG TTA
Gly Pro Asn Thr Glu Phe Ala Leu Ser Leu Leu

AGG AAA AAC ATA ATG ACT ATA ACA ACC TCA AAG GGA GAG
Arg Lys Asn Ile MET Thr Ile Thr Thr Ser Lys Gly Glu

TTC ACA GGG TTA GGC ATA CAT GAT CGT GTC TGT GTG ATA
Phe Thr Gly Leu Gly Ile His Asp Arg Val Cys Val Ile

CCC ACA CAC GCA CAG CCT GGT GAT GAT GTA CTA GTG AAT
Pro Thr His Ala Gln Pro Gly Asp Asp Val Leu Val Asn

```
GGT CAG AAA ATT AGA GTT AAG GAT AAG TAC AAA TTA GTA
Gly Gln Lys Ile Arg Val Lys Asp Lys Tyr Lys Leu Val

GAT CCA GAG AAC ATT AAT CTA GAG CTT ACA GTG TTG ACT
Asp Pro Glu Asn Ile Asn Leu Glu Leu Thr Val Leu Thr

TTA GAT AGA AAT GAA AAA TTC AGA GAT ATC AGG GGA TTT
Leu Asp Arg Asn Glu Lys Phe Arg Asp Ile Arg Gly Phe

ATA TCA GAA GAT CTA GAA GGT GTG GAT GCC ACT TTG GTA
Ile Ser Glu Asp Leu Glu Gly Val Asp Ala Thr Leu Val

GTA CAT TCA AAT AAC TTT ACC AAC ACT ATC TTA GAA GTT
Val His Ser Asn Asn Phe Thr Asn Thr Ile Leu Glu Val

GGC CCT GTA ACA ATG GCA GGA CTT ATT AAT TTG AGT AGC
Gly Pro Val Thr MET Ala Gly Leu Ile Asn Leu Ser Ser

ACC CCC ACT AAC AGA ATG ATT CGT TAT GAT TAT GCA ACA
Thr Pro Thr Asn Arg MET Ile Arg Tyr Asp Tyr Ala Thr

AAA ACT GGG CAG TGT GGA GGT GTG CTG TGT GCT ACT GGT
Lys Thr Gly Gln Cys Gly Gly Val Leu Cys Ala Thr Gly

AAG ATC TTT GGT ATT CAT GTT GGC GGT AAT GGA AGA CAA
Lys Ile Phe Gly Ile His Val Gly Gly Asn Gly Arg Gln

GGA TTT TCA GCT CAA CTT AAA AAA CAA TAT TTT GTA GAG
Gly Phe Ser Ala Gln Leu Lys Lys Gln Tyr Phe Val Glu
```

Protease

AAA CAA

Lys Gln


      6.  DNA representative of genomic RNA of
replicase of HRV type 14 having the nucleotide and
corresponding amino acid sequence:

Replicase

GGC CAA GTA ATA GCT AGA CAT AAG GTT AGG GAG

Gly Gln Val Ile Ala Arg His Lys Val Arg Glu


TTT AAC ATA AAT TCA GTC AAC ACG GCA ACT AAG TCA AAA

Phe Asn Ile Asn Ser Val Asn Thr Ala Thr Lys Ser Lys


TTA CAT CCC AGT GTA TTT TAT GAT GTT TTT CCA GGT GAC

Leu His Pro Ser Val Phe Tyr Asp Val Phe Pro Gly Asp


AAG GAA CCT GCT GTA TTG AGT GAC AAT GAT CCC AGA CTG

Lys Glu Pro Ala Val Leu Ser Asp Asn Asp Pro Arg Leu


GAA GTT AAA TTG ACT GAA TCA TTA TTC TCT AAG TAC AAG

Glu Val Lys Leu Thr Glu Ser Leu Phe Ser Lys Tyr Lys


GGG AAT GTA AAT ACG GAA CCC ACT GAA AAT ATG CTT GTG

Gly Asn Val Asn Thr Glu Pro Thr Glu Asn MET Leu Val


GCT GTA GAC CAT TAT GCA GGG CAA CTA TTA TCA CTA GAT

Ala Val Asp His Tyr Ala Gly Gln Leu Leu Ser Leu Asp


ATC CCC ACT TCT GAA CTT ACA CTA AAA GAA GCA TTA TAT

Ile Pro Thr Ser Glu Leu Thr Leu Lys Glu Ala Leu Tyr

GGA GTA GAT GGA CTA GAA CCT ATA GAT ATT ACA ACC AGT
Gly Val Asp Gly Leu Glu Pro Ile Asp Ile Thr Thr Ser

GCA GGA TTT CCC TAT GTG AGT CTT GGG ATC AAA AAG AGA
Ala Gly Phe Pro Tyr Val Ser Leu Gly Ile Lys Lys Arg

GAC ATT CTG AAT AAA GAG ACC CAG GAC ACA GAA AAG ATG
Asp Ile Leu Asn Lys Glu Thr Gln Asp Thr Glu Lys MET

AAG TTT TAT CTA GAC AAG TAT GGC ATT GAC TTG CCT CTA
Lys Phe Tyr Leu Asp Lys Tyr Gly Ile Asp Leu Pro Leu

GTT ACA TAT ATT AAG GAT GAA TTA AGA AGT GTT GAC AAA
Val Thr Tyr Ile Lys Asp Glu Leu Arg Ser Val Asp Lys

GTC CGA TTA GGG AAA AGT AGA TTA ATT GAA GCC TCC AGT
Val Arg Leu Gly Lys Ser Arg Leu Ile Glu Ala Ser Ser

TTG AAT GAT TCT GTT AAC ATG AGA ATG AAA CTA GGC AAC
Leu Asn Asp Ser Val Asn MET Arg MET Lys Leu Gly Asn

CTT TAC AAA GCA TTC CAT CAA AAT CCC GGT GTT CTG ACT
Leu Tyr Lys Ala Phe His Gln Asn Pro Gly Val Leu Thr

GGA TCA GCA GTG GGT TGT GAT CCT GAT GTG TTT TGG TCT
Gly Ser Ala Val Gly Cys Asp Pro Asp Val Phe Trp Ser

GTC ATC CCT TGC TTA ATG GAT GGG CAC CTG ATG GCA TTT
Val Ile Pro Cys Leu MET Asp Gly His Leu MET Ala Phe

GAT TAC TCT AAT TTT GAT GCC TCT TTG TCA CCA GTT TGG
Asp Tyr Ser Asn Phe Asp Ala Ser Leu Ser Pro Val Trp

0169146

```
TTT GTC TGT CTA GAG AAG GTT TTG ACC AAG TTA GGC TTT
Phe Val Cys Leu Glu Lys Val Leu Thr Lys Leu Gly Phe

GCA GGC TCT TCA TTA ATT CAA TCA ATT TGT AAT ACC CAT
Ala Gly Ser Ser Leu Ile Gln Ser Ile Cys Asn Thr His

CAT ATC TTT AGG GAT GAA ATA TAT GTG GTT GAA GGT GGC
His Ile Phe Arg Asp Glu Ile Tyr Val Val Glu Gly Gly

ATG CCC TCA GGG TGT TCA GGA ACC AGC ATA TTC AAT TCC
MET Pro Ser Gly Cys Ser Gly Thr Ser Ile Phe Asn Ser

ATG ATC AAC AAC ATA ATC ATT AGG ACT TTG ATA TTA GAT
MET Ile Asn Asn Ile Ile Ile Arg Thr Leu Ile Leu Asp

GCA TAT AAA GGA ATA GAT TTA GAC AAA CTT AAA ATC TTA
Ala Tyr Lys Gly Ile Asp Leu Asp Lys Leu Lys Ile Leu

GCT TAC GGT GAT GAT TTG ATT GTT TCT TAT CCT TAT GAA
Ala Tyr Gly Asp Asp Leu Ile Val Ser Tyr Pro Tyr Glu

CTG GAT CCA CAA GTG TTG GCA ACT CTT GGT AAA AAT TAT
Leu Asp Pro Gln Val Leu Ala Thr Leu Gly Lys Asn Tyr

GGA CTA ACC ATC ACA CCC CCA GAC AAA TCT GAA ACT TTT
Gly Leu Thr Ile Thr Pro Pro Asp Lys Ser Glu Thr Phe

ACA AAA ATG ACA TGG GAA AAC TTG ACA TTT TTA AAG AGA
Thr Lys MET Thr Trp Glu Asn Leu Thr Phe Leu Lys Arg
```

```
TAC TTC AAG CCT GAT CAA CAA TTT CCC TTT TTG GTT CAC
Tyr Phe Lys Pro Asp Gln Gln Phe Pro Phe Leu Val His


CCA GTT ATG CCC ATG AAA GAT ATA CAT GAG TCA ATC AGA
Pro Val MET Pro MET Lys Asp Ile His Glu Ser Ile Arg


TGG ACA AAG GAT CCT AAA AAC ACA CAG GAT CAC GTC CGA
Trp Thr Lys Asp Pro Lys Asn Thr Gln Asp His Val Arg


TCA TTA TGC ATG TTA GCA TGG CAC TCA GGA GAA AAA GAG
Ser Leu Cys MET Leu Ala Trp His Ser Gly Glu Lys Glu


TAC AAT GAA TTC ATT CAG AAG ATC AGA ACT ACT GAC ATT
Tyr Asn Glu Phe Ile Gln Lys Ile Arg Thr Thr Asp Ile


GGA AAA TGT CTA ATT CTC CCA GAA TAC AGC GTA CTT AGG
Gly Lys Cys Leu Ile Leu Pro Glu Tyr Ser Val Leu Arg


                    Replicase
AGG CGC TGG TTG GAC CTC TTT
Arg Arg Trp Leu Asp Leu Phe
```

7. A hybridoma cell made by fusing a mammalian myeloma cell to a spleen cell from a mammal immunized with a serotype of the major group of human rhinoviruses of HRV or a protein subunit thereof.

8. A hybridoma cell according to Claim 7 wherein the protein subunit is VP1, VP2 or VP3.

9. A monoclonal antibody to HRV produced by a hybridoma cell of Claim 7.

10. A monoclonal antibody produced by a hybridoma cell made by fusion of a mammalian myeloma cell to a spleen cell from a mammal immunized with a) cells containing receptor sites for serotypes of the major group of human rhinoviruses or with b) cell membranes containing receptor sites for serotypes of the major group of human rhinoviruses or with c) cells and cell membranes containing receptor sites for serotypes of the major group of human rhinoviruses.

11. A monoclonal antibody of Claim 10 wherein the spleen cell is from a mammal immunized with cells containing receptor sites for serotypes of the major group of human rhinoviruses.

12. A monoclonal antibody of Claim 10 wherein the spleen cell is from a mammal immunized with cell membranes containing receptor sites for serotypes of the major group of human rhinoviruses.

13. A monoclonal antibody of Claim 10 wherein the spleen cell is from a mammal immunized with cells and cell membranes containing receptor sites for serotypes of the major group of human rhinoviruses.

14. An antibody according to Claim 10 wherein the mammalian myeloma cell is from a rodent.

15.   An antibody according to Claim 14 wherein the rodent is a mouse.

16.   An antibody according to Claim 10 which prevents attachment of serotypes of the major group of human rhinoviruses to a susceptible mammalian cell.

17.   An antibody according to Claim 16 wherein the antibody prevents attachment for up to about 12 hours.

18.   An antibody according to Claim 10 which is capable of displacing at least some of the serotypes of the major group of human rhinoviruses when the serotypes are bound to a susceptible mammalian cell.

19.   A Fab fragment produced by enzyme digestion of the antibody of Claim 10.

20.   An _in vitro_ composition comprising a continuous hybridoma cell line which secretes the antibody of Claim 10.